(19) Europäisches Patentamt / European Patent Office / Office européen des brevets

(11) **EP 3 019 593 B1**

(12) **FASCICULE DE BREVET EUROPEEN**

(45) Date de publication et mention de la délivrance du brevet:
**17.10.2018 Bulletin 2018/42**

(21) Numéro de dépôt: **14747100.7**

(22) Date de dépôt: **15.07.2014**

(51) Int Cl.:
*C12N 1/12* (2006.01)   *C12N 1/14* (2006.01)
*C12N 1/20* (2006.01)   *C12N 13/00* (2006.01)
*C12P 7/64* (2006.01)   *C12P 23/00* (2006.01)

(86) Numéro de dépôt international:
**PCT/FR2014/051809**

(87) Numéro de publication internationale:
**WO 2015/004403 (15.01.2015 Gazette 2015/02)**

(54) **PROCEDE DE CULTURE CELLULAIRE DECOUPLE**

ENTKOPPELTES ZELLKULTURVERFAHREN

UNCOUPLED CELL CULTURE METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.07.2013 FR 1356918**

(43) Date de publication de la demande:
**18.05.2016 Bulletin 2016/20**

(83) **Déclaration conformément à la règle 32(1) CBE (solution de l'expert)**

(73) Titulaire: **Fermentalg**
**33500 Libourne (FR)**

(72) Inventeurs:
• **GARNIER, Patrice**
**F-33230 Lagorce (FR)**
• **PAGLIARDINI, Julien**
**F-33300 Bordeaux (FR)**

• **CALLEJA, Pierre**
**F-33500 Libourne (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 2 559 342    US-A1- 2009 209 014**

• **MPHO SETLHAKU ET AL: "Improvement in the bioreactor specific productivity by coupling continuous reactor with repeated fed-batch reactor for acetone-butanol-ethanol production", JOURNAL OF BIOTECHNOLOGY, vol. 161, no. 2, 1 octobre 2012 (2012-10-01), pages 147-152, XP055110168, ISSN: 0168-1656, DOI: 10.1016/j.jbiotec.2012.04.004**

EP 3 019 593 B1

**Description**

L'invention concerne un procédé de production de biomasse par culture cellulaire en conditions de mixotrophie, notamment en présence d'un éclairement discontinu et/ou variable de lumière, et/ou en conditions d'hétérotrophie permettant d'obtenir à la fois une augmentation de la concentration cellulaire et de la production de molécules d'intérêt.

Le procédé de production de biomasse selon l'invention comprend la culture des cellules en mode continu en conditions de mixotrophie ou en conditions d'hétérotrophie dans un fermenteur, puis l'alimentation en continu dans des fermenteurs fonctionnant en mode semi-continu par les cellules produites et leur culture en conditions de mixotrophie ou en conditions d'hétérotrophie dans lesdits fermenteurs.

Le procédé est applicable à des cellules et, en particulier, des organismes unicellulaires eucaryotes ou procaryotes. Ce procédé est, en particulier, applicable à des cellules photosensibles, c'est-à-dire capables d'induire une activité métabolique en réponse à une illumination naturelle ou artificielle, dans des conditions de mixotrophie, en particulier des protistes. Le procédé permet d'obtenir conjointement une haute concentration en biomasse et un enrichissement des cellules ainsi cultivées en molécules d'intérêt, notamment en lipides et/ou en caroténoïdes et, plus particulièrement, en acide éicosapentaénoïque (EPA) et/ou en acide docosahexaénoïque (DHA) et /ou en acide arachidonique (ARA), et/ou en acide $\alpha$-linolénique (ALA) et/ou en acide linoléique et/ou acide oléique.

**Préambule**

La production des matières premières par des sources biologiques est devenue de plus en plus importante étant donné le contexte écologique actuel. Des cellules animales et végétales, ainsi que des microorganismes sont utilisés aujourd'hui en tant que producteurs de molécules qui sont utiles pour les industries agro-alimentaire (par exemple, des alcools, des acides organiques, des acides gras, des polysaccharides, des pigments, des acides aminés, des enzymes ou des vitamines), pharmaceutique (antibiotiques, composés à activité pharmacologique, protéines exogènes ou recombinantes), cosmétique (des pigments, des composés organiques comme des acides, des polysaccharides et des acides gras) et pétrochimique (des biofuels ou des produits intermédiaires qui peuvent être convertis en carburant, des lubrifiants et des bioplastiques).

Par exemple, des protistes font l'objet actuellement de nombreux projets industriels car certaines espèces sont capables d'accumuler ou de secréter des quantités importantes de composés valorisables, comme des lipides, notamment des acides gras polyinsaturés.

La production des molécules d'intérêt par des cellules se fait à l'échelle industrielle en général, par des méthodes de fermentation. Pour mettre en oeuvre une fermentation à l'échelle industrielle, plusieurs facteurs doivent être pris en compte, par exemple, pour des protistes, les conditions de culture (autotrophe, mixotrophe ou hétérotrophe) selon la souche, la température, les conditions de lumière et la taille des fermenteurs. Par exemple, les cultures peuvent également être réalisées dans des conteneurs d'un litre, dans un laboratoire, dans des photobioréacteurs, dans des conteneurs de 100 000 litres ou bien dans des bassins ouverts (plusieurs hectares). Toutefois, les dépenses énergétiques et autres ressources, telles que la main d'oeuvre et la facilité de poursuivre la culture, doivent être prises en compte en développant des conditions de culture idéales.

En tout état de cause, il est souhaitable que les cellules soient cultivées dans des conditions optimales pour augmenter le rendement des molécules à produire.

Il existe trois types de procédés de fermentation couramment utilisés dans le domaine de la production de biomasse, à savoir,

-    le mode discontinu (en anglais « batch ») :
La cuve est remplie par le milieu de culture stérilisé, puis l'inoculum. La fermentation se déroule ensuite sans addition majeure supplémentaire de milieu (principalement les correctifs pH et antimousse). Le volume reste constant et la productivité de biomasse est relativement faible. En fin de fermentation, le fermenteur est vidé et son contenu est remplacé.

-    le mode semi-continu (en anglais « fed-batch ») :
Ce procédé de production repose sur l'alimentation progressive de substrat nutritif lors de la culture. Ce mode est généralement utilisé pour atteindre une densité cellulaire élevée dans le fermenteur.

-    le mode continu (en anglais « continuous ») :
Dans ce procédé, on utilise un fermenteur couramment appelé « chemostat » dans lequel un milieu de culture frais est ajouté en permanence, tandis que le liquide de culture est soutiré en permanence pour garder le volume de culture constant.

Le taux de croissance des cellules peut être contrôlé en modifiant le débit d'alimentation en matière nutritive tout en respectant les limites physiologiques de chaque type de cellule ou souche (en fonction de son taux de croissance spécifique maximal ($\mu$max) dans les conditions spécifiques d'un procédé).

Le mode continu permet la culture des microorganismes dans un état d'équilibre physiologique donné correspondant à un équilibre entre la croissance de la biomasse et la production de métabolite(s) d'intérêt.

Dans l'état d'équilibre, les cellules croissent à une vitesse constante et tous les paramètres de culture demeurent constants (pH, volume, concentration en oxygène dissous, les concentrations en éléments nutritifs et en produits, densité cellulaire, etc..).

Le mode continu présente, en particulier, les avantages suivants :

- le coût de production global de biomasse est diminué (moins d'opérations de nettoyage, stérilisation, culture, immobilisation d'équipements au niveau de la fermentation);

- la productivité (quantité de biomasse produite par heure) est augmentée;

- la récupération de la biomasse de manière continue permet de faciliter les opérations en aval tout en optimisant le dimensionnement des équipements.

Cependant, les procédés continus présentent également des inconvénients, notamment lorsque la production de la molécule d'intérêt est dissociée ou partiellement dissociée de la phase de croissance cellulaire. Ainsi, lorsqu'une substance d'intérêt est produite en dehors de cette phase de croissance optimale (par exemple en fin de culture, pendant la phase stationnaire de la culture), il est plus difficile d'obtenir des performances compatibles avec les intérêts économiques.

D'autres contraintes liées au mode continu sont :

- la gestion des effluents en plus grande quantité (concentration en biomasse plus faible),

- la perte de matières premières non consommées (substrats résiduels) pouvant également avoir un impact sur les processus en aval (extraction, séchage...).

Le brevet US 6156570 décrit un procédé de culture cellulaire en suspension, en conditions hétérotrophes, dans lequel la culture est initiée en mode semi-continu et poursuivie en mode continu. Ce procédé permet de modifier le métabolisme énergétique cellulaire, résultant en une faible production de lactate, ce qui permet de maintenir une forte concentration cellulaire. Les cellules utilisées sont de préférence des cellules de mammifères, ou de source animale autre (insectes, poissons etc.).

La demande WO2003/020919 concerne un système à plusieurs unités pour la culture de cellules animales ou végétales à haute densité cellulaire, en suspension, en vue de l'obtention de produits biologiques. Ce procédé utilise notamment un fermenteur de pré-culture en mode semi-continu et un fermenteur de culture en mode continu. Le procédé décrit repose sur l'arrangement spatial des différentes unités permettant de les mettre en oeuvre de manière synergique.

La demande US2009/0263889 concerne un procédé de culture en conditions hétérotrophes de protistes, en particulier des genres *Schizochytrium* ou *Thraustochytrium,* pour la production de lipides cellulaires tels que le DHA. La culture est initiée en mode discontinu et poursuivie en mode continu, ou comprend une étape intermédiaire de culture en mode semi-continu.

La demande WO2012/074502 décrit des procédés et des systèmes de culture de différents microorganismes photo-autotrophes, notamment des algues, des diatomées, des cyanobactéries, des photobactéries ou des plantes, dans lesquels un agent de stimulation « biotique » (issu d'un autre organisme) ou « abiotique » (inorganique ou produit par d'autres moyens) est utilisé pour générer ou accroître la production de métabolites. Cet agent de stimulation peut être utilisé en combinaison avec une illumination. Ce procédé combine des conditions de culture photo-autotrophes et mixotrophes, ou bien photo-autotrophes et hétérotrophes.

La demande US 2009/209014 décrit une culture de *Schizochitrium* en mode hétérotrophe comprenant plusieurs étapes, dont une combinaison continu/discontinu en mode hétérotrophe, c'est à dire sans éclairage.

On a maintenant trouvé qu'un procédé de production de biomasse comprenant la culture des cellules en mode continu en conditions de mixotrophie ou hétérotrophie dans un fermenteur (étape de « croissance »), puis l'alimentation en continu et alternativement dans des fermenteurs fonctionnant en mode semi-continu par les cellules produites et leur culture en conditions de mixotrophie ou hétérotrophie dans lesdits fermenteurs (étape de « maturation »), permettait d'obtenir à la fois un accroissement de la productivité de biomasse et de molécules d'intérêt.

Avantageusement, un tel procédé de production présente notamment les avantages suivants :

- la biomasse produite en mode continu a un taux de croissance important;

- la masse cellulaire est significativement augmentée par accumulation de molécules d'intérêt lors de la culture en mode semi-continu;

- les phases de croissance et de production de molécules d'intérêt sont séparées physiquement, ce qui permet également une optimisation des paramètres de culture pour chacune des étapes (lumière, température, pH, $pO_2$, agitation, etc.);

- la phase de croissance en mode continu alimente ensuite des fermenteurs de maturation et permet de réduire considérablement les phases d'arrêts de production liés au nettoyage et la stérilisation qui sont rencontrés lors de cultures discontinues (« Batch ») ou semi-continues (« Fed-Batch »);

- en comparaison à un mode de production continu, le procédé de l'invention permet d'obtenir une composition optimale de la biomasse produite. C'est-à-dire que l'utilisation de paramètres physico-chimiques lors de la phase de maturation qui sont orientés pour l'accumulation de la molécule d'intérêt permet d'en améliorer considérablement la productivité en comparaison à une production en mode continu;

- Selon le procédé de l'invention, il y a moins d'effluents en comparaison avec un mode continu car la phase de maturation permet d'augmenter le rendement de biomasse au litre et donc de réduire la quantité d'effluents à retraiter pour une quantité de biomasse équivalente.

Les molécules d'intérêt dont la production est recherchée sont, en particulier, des pigments, tels que la fucoxanthine, l'astaxanthine, la zéaxanthine, la canthaxanthine, l'échinénone, le bêta-carotène et la phoenicoxanthine, des (exo-) polysaccharides, des acides aminés, des vitamines, des lipides, notamment, des acides gras, et plus particulièrement des acides gras polyinsaturés. Parmi ceux-ci, certains hautement insaturés (AGHI) de la série des oméga-3 (« polyunsaturated fatty acids » en anglais, PUPA-$\omega$3), en particulier l'acide éicosapentaénoïque (EPA ou C20:5 $\omega$3) ainsi que son précurseur, l'acide linolénique (ALA ou C18 :5 $\omega$3.) et l'acide docosahexaénoïque (DHA ou C22:6 $\omega$3), et de la série des oméga-6 (PUFA-$\omega$6), en particulier l'acide arachidonique (ARA ou AA ou encore acide eicosatétraénoïque C20:4 $\omega$6), ainsi que son précurseur, l'acide linoléique (C18 :21 $\omega$6), ont une importance nutritionnelle reconnue et présentent de fortes potentialités en terme d'applications thérapeutiques. Les acides gras mono-insaturés, notamment de l'acide oléique, présentent un intérêt aussi.

Les huiles de poissons, issues de l'industrie de la pêche, sont actuellement la principale source commerciale de ce type d'acides gras. Toutefois, alors que ces huiles trouvent de nouvelles applications (complément alimentaire en aquaculture, intégration dans les margarines), les ressources halieutiques marines se raréfient du fait d'une activité de pêche intensive.

De nouvelles sources de ces acides gras tels que l'acide oléique, l'EPA, le DHA, l'ALA et l'ARA doivent donc être recherchées afin de répondre, dans le futur, à la demande croissante du marché pour ce type d'acides gras polyinsaturés.

Des acides gras à chaîne courte (C1 à C7) par exemple les acides acétique, propanoïque et butyrique, ou des acides gras à chaîne moyenne (C8 à C12), par exemple l'acide laurique, sont aussi des molécules issues de microorganismes et en particulier des protistes.

Par ailleurs, les caroténoïdes, tels que l'astaxanthine, la zéaxanthine, la canthaxanthine, l'échinénone, le bêta-carotène et la phoenicoxanthine, la lutéine ou la fucoxanthine, sont également des molécules d'intérêt produites par des microorganismes. Ils sont généralement utilisés en tant que pigments, mais ils ont aussi un rôle important pour la santé de l'homme en tant qu'agents antioxydants. Enfin, ils présentent la capacité de stimuler le système immunitaire.

La lutéine, ainsi que la zéaxanthine en quantités beaucoup moins importantes, sont les seuls caroténoïdes qui sont absorbés dans le sang après ingestion et accumulés dans la rétine humaine. La lutéine est associée à une possible réduction des risques liés aux lésions oculaires et cutanées causées par la lumière bleue. Elle est notamment associée à la prévention de la dégénérescence maculaire liée à l'âge (DMLA).

Par ailleurs, des molécules connues en tant que « Mycosporine like amino acids » ou « MAAs » en anglais ou acides aminés de type mycosporine en français, sont des molécules d'intérêt produites par des microbes tels que des bactéries marines, des cyanobactéries, des champignons et divers autres organismes marins.

Par ailleurs, les exo-polysaccharides produits par certains microorganismes sont des molécules d'intérêt car ils peuvent entrer dans la composition de certaines préparations agroalimentaires comme agent texturant ; ou encore comme composant de certaines formulations cosmétiques, par exemple comme agent matifiant ou texturant.

Le procédé de l'invention vise la culture de toute sorte de cellules, en particulier la culture des protistes pour la production des acides gras et/ou des pigments à l'échelle industrielle.

La plupart des espèces de protistes rencontrées dans l'eau douce ou les océans sont généralement autotrophes, c'est-à-dire qu'elles ne peuvent croître que par photosynthèse. Pour celles-ci, la présence dans leur milieu de substrats carbonés organiques ne leur est pas favorable et n'améliore pas leur croissance. Cependant, un certain nombre d'espèces de protistes, de familles et d'origines très diverses, s'avèrent ne pas être strictement autotrophes. C'est ainsi que certaines d'entre elles, dites hétérotrophes, sont capables de se développer en l'absence totale de lumière, par le biais d'un métabolisme oxydatif (fermentation ou respiration), c'est-à-dire en exploitant un substrat carboné organique, comme les sucres, comme seule source de carbone et d'énergie.

D'autres espèces de protistes, pour lesquelles la photosynthèse reste indispensable à leur développement, sont capables à la fois de tirer parti de la photosynthèse et de la matière organique présente dans leur milieu. Ces espèces intermédiaires, dites mixotrophes, peuvent exploiter la présence de lumière et de substrats carbonés organiques.

Cette particularité des algues dites « mixotrophes » semble être liée à leur métabolisme, qui leur permet d'opérer simultanément photosynthèse et métabolisme oxydatif. Les deux types de métabolisme coexistent avec un effet global positif sur la croissance des algues [Yang, C. et al. (2000) ; Biochemical Engineering Journal, 6 :87-102].

Il existe d'autres organismes considérés comme mixotrophes. Par exemple, la bactérie *Paracoccus pantotrophus,* des protistes tels qu'*Euglena,* et la plante *Dionaea muscipula,* sont capables de tirer de l'énergie de la lumière. La méthode de culture selon l'invention est applicable aux cellules des organismes dites « mixotrophes ».

Parmi les espèces mixotrophes, nous pouvons également citer le cas des espèces « photo-hétérotrophes », qui se développent uniquement par le biais d'un métabolisme oxydatif (fermentation ou respiration), mais dont la lumière a un impact sur la cellule via des photorécepteurs, comme par exemple le phototropisme (capacité à s'orienter par rapport à la lumière), le déclenchement de cycles de reproduction sexuée ou plus généralement lorsque la lumière engendre une modification du métabolisme. Ces mécanismes sont connus chez de nombreux organismes comme par exemple les champignons filamenteux d'origine marine ou encore des protistes.

Dans la présente invention, par cellule, organisme ou microorganisme « mixotrophe », on entend des cellules, organismes ou microorganismes capables de croître en utilisant la lumière et une source carbonée organique, et également des microorganismes et cellules également appelés « photo-hétérotrophes » qui utilisent la lumière pour une autre fonction que la croissance, comme par exemple la production des molécules comme des caroténoïdes. Ainsi, les cellules « mixotrophes » sont capables d'induire une activité métabolique en réponse à une illumination naturelle ou artificielle, dans des conditions de mixotrophie.

En tout état de cause, il est souhaitable que les microorganismes, ou cellules, soient cultivés dans des conditions optimales pour augmenter le rendement des molécules d'intérêt, en particulier, de(s) (l')acide(s) gras et/ou des pigments à produire.

En particulier, il serait souhaitable de pouvoir obtenir des rendements en biomasse, en lipides et en caroténoïdes plus importants que ce qui est décrit dans l'art antérieur pour une exploitation industrielle plus efficace et plus rentable. Ainsi, il est préférable d'avoir un rendement le plus élevé possible (par exemple au-delà de 80 g/l de matière sèche, plus de 30% d'acides gras en poids par rapport au poids total de la matière sèche et plus de 0,1 % en poids de caroténoïdes sur le poids total de matière sèche).

**Description détaillée**

L'invention concerne donc, selon un premier aspect, un procédé de production de biomasse de cellules d'organismes unicellulaires eucaryotes capables de croître en utilisant la lumière et une source carbonée organique, caractérisé en ce qu'il consiste en :

a) la culture des cellules en mode continu en conditions de mixotrophie ou hétérotrophie dans un fermenteur, dit « fermenteur de tête », puis
b) l'alimentation en continu de *n* fermenteurs, dits « fermenteurs de maturation » fonctionnant en mode semi-continu, *n* étant un nombre entier supérieur à 2, de préférence de 2 à 10, par les cellules produites dans l'étape a) et leur culture en conditions de mixotrophie

et en ce que la culture des cellules en conditions de mixotrophie s'effectue dans des conditions d'éclairement discontinu et/ou variable au cours du temps

Selon un mode de réalisation, l'invention concerne un procédé de culture qui comprend une étape de croissance en mixotrophie ou hétérotrophie et une étape d'accumulation de molécules d'intérêt dans des conditions de mixotrophie.

Par « conditions de mixotrophie », on entend une culture avec un apport de lumière et un apport en substrat carboné organique.

Par « conditions en hétérotrophie », on entend un apport en substrat carboné organique et dans l'obscurité.

Par « protistes », on entend tous les microorganismes unicellulaires eucaryotes. Les microalgues (*Chlorella, Tetraselmis, Nitzschia...etc.*), les champignons unicellulaires (*Schizochytrium, Aurantiochytrium* etc.) ou les flagellés hétérotrophes (*Crypthecodinium* etc.) font partie du groupe des protistes.

Par « souche », on entend non seulement les souches naturelles, mais également les mutants desdites souches naturelles.

Par « mutants », on entend un organisme dérivé de la souche originelle, dont le patrimoine génétique a été modifié soit par un processus naturel, soit par des méthodes physico-chimiques connues par l'homme du métier pouvant générer des mutations aléatoires (UV etc.,), soit par des méthodes de génie génétique.

L'étape a) est la phase de croissance des cellules permettant d'atteindre une population importante. En général, un milieu de culture (solution d'alimentation des cellules) spécifique est choisi pour atteindre ce but, en fonction du type de cellules cultivées, et la/les molécule(s) d'intérêt à produire. Par exemple, dans le cas des protistes, le milieu de culture est enrichi avec une solution d'alimentation contenant des macroéléments, des oligoéléments et des vitamines. Le milieu contient un substrat carboné organique, qui permet la culture en mode hétérotrophe ou mixotrophe.

L'étape b) est une phase d'accumulation de molécules d'intérêt. De préférence, cette phase utilise une seconde solution d'alimentation permettant d'orienter préférentiellement le métabolisme vers l'accumulation de cette molécule d'intérêt. Par exemple, en cas de production de lipides par des protistes, cette orientation peut se faire par l'augmentation du rapport de la concentration du carbone sur celle de l'azote assimilable (rapport C/N).

En pratique, un premier fermenteur fonctionnant en mode continu permet la croissance (l'étape a)) des cellules, et le flux sortant (ou soutirage) de ce fermenteur de croissance alimente des cuves de maturation où la biomasse s'enrichit en molécules d'intérêt (l'étape b)).

Le procédé peut être décrit en tant qu'un procédé dit « découplé » car les phases de croissance selon l'étape a) et de maturation selon l'étape b) sont séparées physiquement et temporellement.

L'étape a) de culture des cellules en mode continu du procédé de production de biomasse selon l'invention, ou « étape de croissance », peut être mise en oeuvre dans un fermenteur connu de l'homme de métier, comme par exemple, le « chemostat ».

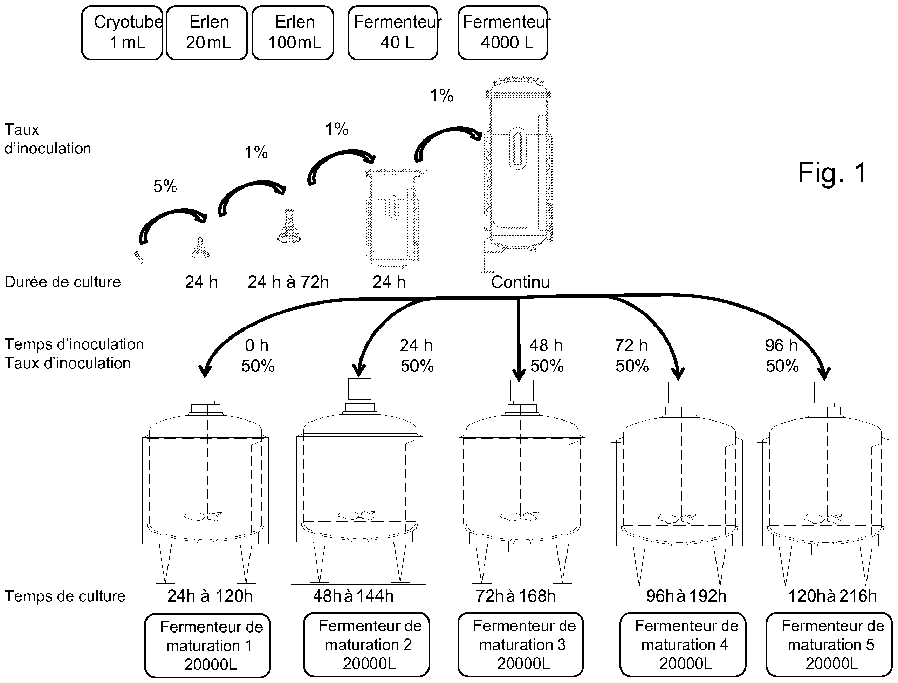

Le démarrage de la culture de l'étape a) peut se faire de manière classique (voir Figure 1). Des étapes de pré-culture peuvent être effectuées en amont, de manière à obtenir la concentration cellulaire voulue pour débuter la production de biomasse en continu, à savoir entre 2 et 50 g/L, de préférence entre 5 et 40 g/L.

En général, la montée en échelle à partir du cryotube se fait en plusieurs étapes de pré-culture. Par exemple, elle peut se faire en trois étapes, deux étapes en Erlenmeyer (préculture 1 et préculture 2), et une étape en fermenteur (pré-fermenteur). La croissance en Erlenmeyer se déroule en mode discontinu (« batch »). En général, la croissance en pré-fermenteur se fait également en mode discontinu.

Par exemple, la culture peut passer progressivement d'une culture en Erlenmeyer de 20 mL à une culture en Erlenmeyer de 2 L, puis à une culture en fermenteur (pré-fermenteur) en mode discontinu (« batch »), puis, à une culture dans un fermenteur en mode continu (« fermenteur de tête »). Par exemple, le fermenteur en mode discontinu a un volume de 40 L et le fermenteur de tête a un volume de 4000 L.

Le démarrage de la culture dans le fermenteur de tête a une phase de mise en place durant laquelle le régime continu n'est pas atteint, c'est-à-dire que la croissance des cellules n'est pas stabilisée, ou n'est pas en phase avec le débit d'alimentation en milieu frais. Il s'agit d'une phase « d'atteinte de l'équilibre » que l'on pourrait aussi appeler « phase de mise en régime permanent ». Cette « phase de mise en régime permanent » a une durée qui dépend de la vitesse de croissance des microorganismes cultivés ou plus précisément du taux de croissance spécifique ou $\mu$max. A titre exemple, cette durée peut être comprise entre 5 et 200 heures, de préférence entre 10 et 100 heures.

De manière générale, une fois le régime permanent atteint, un débit de soutirage du fermenteur de tête vers le ou les fermenteur(s) de maturation est mis en place à un débit équivalent au débit d'alimentation en milieu de culture frais. Ce soutirage alimente un premier fermenteur de maturation jusqu'à ce que le volume souhaité soit transféré, puis le soutirage est réorienté vers une autre cuve de maturation. L'homme de métier saura déterminer le volume d'inoculum nécessaire selon le type de cellules à cultiver, la molécule d'intérêt à produire et les capacités des cuves de maturation selon un mode de réalisation. Ce volume d'inoculum peut être compris entre 10 et 90 % de la capacité du fermenteur de maturation, en fonction des cellules cultivées ; par exemple environ 20 %, 30 %, 40 %, 50 %, 60 %, 70 %, ou 80 % de la capacité du fermenteur de maturation.

Le soutirage vers les fermenteurs de maturation est fait de manière conventionnelle en utilisant des équipements connus.

De manière générale, un milieu de culture différent de celui utilisé pendant l'étape a) est employé pendant la phase de maturation. Selon des modes de réalisation différents, ce milieu de culture est apporté dans le fermenteur de maturation simultanément, ou préalablement, ou ultérieurement à l'inoculum qui provient du soutirage du fermenteur de tête.

Le volume de la culture en fermenteur de l'étape a) est déterminé en fonction du volume final de culture souhaité pour les cultures en fermenteurs de maturation. En général, le fermenteur de tête a un volume de 5 à 100 %, de préférence 10 % à 50 % et plus préférentiellement environ 20 % du volume du fermenteur de maturation qu'il alimente. Par exemple, un fermenteur de tête ayant un volume de 4000 L peut alimenter un fermenteur de maturation de 20 000 L. Si les n fermenteurs de maturation ont des volumes différents, c'est le volume de fermenteur le plus important qui va déterminer le volume du fermenteur en tête.

Le milieu de culture utilisé dans l'étape a) est choisi selon les caractéristiques des cellules cultivées.

Un milieu minimum ou un milieu nutritif enrichi peut être utilisé et on peut citer à titre d'exemple le milieu de culture Verduyn pour la croissance de protistes.

Pour la culture de microorganismes en général ou de protistes, le milieu de culture pour l'étape a) contient en général une source d'azote et de phosphate, ainsi qu'un substrat carboné organique. Ce dernier permet une culture en mode mixotrophe.

Un exemple de ce type de milieu est le milieu Verduyn modifié (sels marins 15 g/L, $(NH_4)_2SO_4$ 3g/L, $KH_2PO_4$ 1 g/L, $MgSO_4 \cdot 7H_2O$, 0,5 g/L, $Na_2EDTA$ 24 mg/L, $ZnSO_4 \cdot 7H_2O$ 3 mg/L, $MnCl_2 \cdot 2H_2O$ 3 mg/L, $Na_2MoO_4 \cdot 2H_2O$ 0,04 mg/L, $FeSO_4 \cdot 7H_2O$ 10mg/L, pantothénate 3,2 mg/L, hydrochlorure de thiamine 9,5 mg/L, vitamine B12 0,15 mg/L, antimousse 0,1mL/L) dans lequel est ajouté un substrat carboné organique.

Un autre exemple d'un milieu de culture est : sels marins 25 g/L, $KNO_3$ 3g/L, $NaH_2PO_4$ 1 g/L, $MgSO_4 \cdot 7H_2O$, 2,5 g/L, $Na_2EDTA$ 24 mg/L, $Na_2SiO_3$ $9H_2O$ 0,3g/L, $ZnSO_4 \cdot 7H_2O$ 1 mg/L, $MnCl_2 \cdot 2H_2O$ 1 mg/L, $Na_2MoO_4 \cdot 2H_2O$ 0,04 mg/L, $FeSO_4 \cdot 7H_2O$ 10mg/L, extraits de levure 2g/L, Biotine 0,15mg/L, hydrochlorure de thiamine 9,5mg/L, Vitamine B12 0,15mg/L, antimousse 0,1 mL/L) dans lequel est ajouté un substrat carboné organique.

Selon un mode de réalisation de l'invention, le substrat carboné organique a une concentration de 5 mM à 1,1 M (de 0,9 à 200 g/L), de préférence de 50 mM à 800 mM (de 9 à 144 g/L).

Ce substrat carboné organique comprend préférentiellement, sous forme pure ou en mélange : du glucose, des dérivés de cellulose, du lactate, de l'amidon, du lactose, du saccharose, de l'acétate et/ou du glycérol.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en mixotrophie des cellules selon l'invention.

Du substrat additionnel est ajouté au milieu de culture pendant le procédé de culture pour maintenir une concentration suffisante pour la culture. L'homme du métier sait comment s'assurer que le niveau de substrat carboné organique est maintenu.

De préférence, le substrat carboné organique comprend du glucose, du glycérol, dans une concentration équivalente ou supérieure à 300 mM.

A titre exemple, la culture selon l'étape a) des souches de protistes du genre *Schizochytrium* ou *Aurantiochytrium* pour la production de DHA peut se faire en milieu de culture salin (sel marin 10-20 g/L, de préférence 15 g/L) avec une concentration de glucose de 5 à-200 g/L, de préférence 30 à 100 g/L et plus préférentiellement de 30 à 60 g/L. Selon un mode de réalisation, le milieu de culture contient également des macroéléments, tels que des sels de magnésium et des sels de potassium, à une concentration de 1 à 50 g/L, de préférence 2 à 25 g/L. Selon un mode de réalisation, le milieu contient des sels d'ammonium, de préférence à une concentration de 1,5 à 7,5 g/L. Il contient aussi des oligoéléments utilisés de manière conventionnelle pour la culture des protistes, tels que des sels de manganèse, de zinc, de cobalt, de molybdène et de cuivre, de nickel et de fer. Des valeurs typiques pour la concentration de ces oligoéléments sont de 0,02 à 15 mg/L pour chaque oligoélément. Par exemple, les sels de magnésium et de zinc peuvent être présents à 2-4mg/L, les sels de cobalt et de molybdène peuvent être présents en faible quantité, telle que 0,01-0,04 mg/L, de préférence entre 0,02-0,03 mg/L. Les sels de cuivre et de nickel peuvent être présents à 1- 4 mg/L, de préférence à 1-2 mg/L. En général, les sels de fer sont présents à un taux plus important par rapport à d'autres sels, par exemple à un niveau de 8-15 mg/L.

Selon un autre mode de réalisation, les sels de magnésium sont présents à 70-300 mg/L. Selon un mode de réalisation, le milieu comprend également des vitamines telles que thianine, vitamine B12, panthoténate et éventuellement un agent stabilisant.

Selon un mode de réalisation de l'invention adapté pour atteindre des très hautes densités d'Aurantiochytrium (1 à 3.10$^9$ cellules/ml) cellulaires, le milieu de culture nommé « Milieu Pied » du Tableau 1 ci-dessous peut être utilisé.

**Tableau 1 :** Exemple de milieux de culture utiles pour la culture des souches d'Aurantiochytrium, selon l'étape a) du procédé de culture.

|  | Milieu Pied | Solution alimentation |
|---|---|---|
| **Source carbone** | 1 à 1,6 CMol | 3,7 CMol |
| **NH4$^+$** | 1,5 à 7,5 g/L | 5 à 10 g/L |
| **Sel marin** | 5 à 30 g/L | 10 à 30 g/L |

(suite)

| | Milieu Pied | Solution alimentation |
|---|---|---|
| **Ca** | 25 à 70 mg/L | 100 à 200 mg/L |
| **Mn** | 1 à 10 mg/L | 4 à 20 mg/L |
| **Zn** | 0,1 à 1 mg/L | 0,5 à 2 mg/L |
| **S** | 0,5 à 1,5 g/L | 2 à 4 g/L |
| **Co** | 0,1 à 1 mg/L | 0,5 à 2 mg/L |
| **Mo** | 0,01 à 0,5 mg/L | 0,05 à 1,5 mg/L |
| **Cu** | 0,05 à 0,3 mg/L | 0,25 à 0,8 mg/L |
| **Ni** | 0,02 à 0,2 mg/L | 0,1 à 0,5 mg/L |
| **K** | 0,5 à 2 g/L | 3 à 5 g/L |
| **Mg** | 70 à 300 mg/L | 250 à 750 mg/L |
| **P** | 0,3 à 1 g/L | 1,3 à 2,5 g/L |
| **Fe** | 5 à 10 mg/L | 15 à 25 mg/L |
| **Thiamine** | 15 à 50 mg/L | 50 à 150 mg/L |
| **Vitamine B12** | 0,2 à 0,7 mg/L | 0,7 à 2 mg/L |
| **Panthoténate** | 0,05 à 0,15 mg/L | 15 à 45 mg/L |
| **Agent stabilisant (type EDTA)** | A fixer par le préparateur | A fixer par le préparateur |

Le « milieu Pied » correspond au milieu qui peut être utilisé pour le démarrage de la culture en continu. Ensuite « la solution d'alimentation » peut être utilisée pour alimenter le fermenteur en continu.

De manière générale, lorsque la concentration en biomasse dans le fermenteur de tête a atteint un équilibre, par exemple pour des protistes, bactéries et levures, entre environ 5 et 100 g/L, de préférence entre environ 10 et 70 g/L en concentration de biomasse, le soutirage du fermenteur de tête sert à alimenter de manière continue et successive les différents fermenteurs de maturation fonctionnant en mode semi-continu. La culture des cellules est ainsi maintenue en conditions de mixotrophie ou hétérotrophie dans lesdits fermenteurs (étape de « maturation »), en vue de la production de molécules d'intérêt. Ainsi, de manière générale, l'alimentation des fermenteurs de maturation selon l'étape b) démarre quand le fermenteur de tête est en régime permanent à une densité cellulaire stable. Par exemple, pour la culture des protistes, cette densité cellulaire pourrait être entre $10^7$ et $10^9$ cellules par mL. Pour des levures, cette densité cellulaire pourrait être entre $10^8$ et $10^9$ cellules par mL. Pour des bactéries, cette densité cellulaire pourrait être entre $10^9$ et $10^{10}$ cellules par mL.

L'alimentation des fermenteurs de maturation selon l'étape b) se fait (en général) de manière successive, c'est-à-dire que l'on alimente un fermenteur n de maturation avec le soutirage du fermenteur de tête avant de passer au fermenteur de maturation suivant, n+1.

Le volume d'inoculum utilisé pour inoculer le fermenteur de maturation est en général dépendant de la cellule cultivée et de la ou des molécule(s) d'intérêt à produire et du volume nécessaire dans le fermenteur de maturation avant de passer à l'étape de production de la molécule d'intérêt. Par exemple, dans le cas de protistes, levures et bactéries, le volume peut représenter entre 10 et 70 % du volume final de la suspension cellulaire dans le fermenteur de maturation, préférentiellement entre 20 et 60 %, plus préférentiellement entre 30 et 50 %.

De manière générale, une fois que le volume d'inoculum souhaité a été atteint dans le fermenteur de maturation n, on alimente le deuxième (n+1) fermenteur de maturation (ou « maturateur »), puis on alimente le troisième (n+2) fermenteur de maturation, etc.

Selon un mode de réalisation, la culture dans le fermenteur de maturation est arrêtée, puis le fermenteur est vidé quand la production de la ou des molécule(s) d'intérêt a atteint le niveau désiré, par exemple, dans le cas de protistes du genre *Aurantiochytrium,* lorsque le titre en DHA est entre 5 et 30 g/L et le titre en pigment caroténoïde entre 0,1 et 10 mg/g de biomasse sèche.

La vidange se fait de manière conventionnelle vers des outils permettant le traitement de la biomasse en vue de l'extraction de la ou des molécule(s) d'intérêt. Après avoir été vidé, le fermenteur de maturation est nettoyé, généralement par un équipement spécifique qui est intégré aux cuves des fermenteurs, puis est stérilisé.

Par exemple (voir le Tableau 2 et le schéma selon la Figure 1), avec un temps de culture dans les fermenteurs de maturation de 72h, le premier jour, J, le fermenteur de maturation 1 est rempli et les autres fermenteurs de maturation sont en attente; à J+ 1, le fermenteur de maturation 2 est rempli (culture N+2) et le fermenteur de maturation 1 est en cours de culture; à J+ 2, le fermenteur de maturation 3 est rempli et les fermenteurs de maturation 1 et 2 sont en cours de culture; à J+ 3, le fermenteur de maturation 1 est nettoyé, le fermenteur de maturation 4 est rempli et les fermenteurs de maturation 2 et 3 sont en cours de culture.

**Tableau 2**

| | Fermenteur de maturation 1 | Fermenteur de maturation 2 | Fermenteur de maturation 3 | Fermenteur de maturation 4 |
|---|---|---|---|---|
| **Jour J** | Culture N | Attente | Attente | Attente |
| **Jour J+1** | Culture N | Culture N+1 | Attente | Attente |
| **Jour J+2** | Culture N | Culture N+1 | Culture N+2 | Attente |
| **Jour J+3** | Nettoyage et stérilisation | Culture N+1 | Culture N+2 | Culture N+3 |
| **Jour J+4** | Culture N+4 | Nettoyage et stérilisation | Culture N+2 | Culture N+3 |
| **Jour J+5** | Culture N+4 | Culture N+5 | Nettoyage et stérilisation | Culture N+3 |

Le nombre de fermenteurs de maturation alimentés par le fermenteur de tête est supérieur à 2, et, de manière générale, ce nombre est limité seulement par l'espace disponible pour stocker lesdits fermenteurs et leurs équipements annexes. En général, le nombre de fermenteurs de maturation est de 2 à 10, de préférence de 2 à 6.

Selon un mode de réalisation de l'invention, le temps de culture de maturation varie entre 10 et 200 heures, de préférence entre 20 et 100 heures. Le cycle illustré dans le tableau varie en fonction de ce temps. Par exemple, pour des cellules végétales, le temps de culture de maturation peut varier entre 48 et 200 heures.

Pour la culture des protistes et des levures, cette étape de culture en mode semi-continu est de manière générale effectuée pendant 24 à 200 h dans chaque fermenteur, de préférence pendant 48 h à 96 h, plus préférentiellement 72 h à 96 h.

La composition du milieu de culture pour l'étape b) permet de maintenir une croissance résiduelle, tout en favorisant l'accumulation des molécules d'intérêt. Par « croissance résiduelle », on entend une multiplication des cellules à un taux de croissance compris entre 1 et 90 % du taux de croissance de l'étape a) mais qui permet d'améliorer la productivité en biomasse et en molécule(s) d'intérêt.

Par exemple, pour la production des lipides par des protistes, le taux de croissance en culture continue est de 0,05 à 0,20 h⁻¹, de préférence de 0,10 h⁻¹.

Le taux de croissance caractérise l'accroissement de la population au cours du temps. Le taux de croissance ($\mu$) est calculé selon la formule :

$$\mu = (\ln N2 - \ln N1) / (t2 - t1)$$

où N1 = quantité de biomasse au temps t1, N2 : quantité de biomasse au temps t2. La quantité de biomasse peut être exprimée en densité optique, densité cellulaire ou masse sèche. Les temps t1 et t2 sont exprimés en heure. La valeur de $\mu$ est exprimée en h⁻¹ (1/heure).

Le milieu de culture utilisé pour la fermentation selon l'étape b) de culture en semi-continu est choisi selon la nature des cellules cultivées. De préférence, ce milieu de culture permet d'orienter préférentiellement le métabolisme des cellules cultivées vers l'accumulation d'une ou plusieurs molécule(s) d'intérêt. Pour certaines cellules, la limitation d'un élément dans le milieu de culture est une méthode qui permet de favoriser l'accumulation d'une molécule. A titre d'exemple, nous pouvons citer les protistes qui peuvent augmenter l'accumulation des lipides lors d'une limitation en azote. Par limitation en azote, on entend une concentration en azote insuffisante dans le milieu de culture pour assurer une croissance normale des cellules, et qui se traduit par une modification du métabolisme favorisant l'accumulation du carbone sous forme de lipides. Cette modification du métabolisme peut également être obtenue en augmentant le rapport C/N (rapport de la concentration de carbone sur celle d'azote assimilable).

Toutefois, le milieu de culture contient une source de carbone organique, qui permet une croissance en mixotrophie ou hétérotrophie. Typiquement, pour une culture de protiste, le carbone organique est présent à une concentration de 10 à 90 g/l (de 55mM à 500mM).

En général, la culture selon l'étape b) de maturation des protistes pour la production de lipides ou de pigments peut se faire en milieu de culture salin (sel marin 10-30 g/L) comprenant environ 20 à 200 g/L (0,1 à 1,1M) de substrat carboné organique, environ 1 à 15 g/L d'ammonium et environ 0,9 à 3,5 g/L de phosphate. A titre d'exemple pour la production de DHA par la souche *Aurantiochytrium,* le milieu de culture (reconstitué à partir du volume de la suspension cellulaire issue du fermenteur de tête et du volume de solution d'alimentation concentrée) comprend de 100 à 200 g/L de glucose, environ 5 à 10g/L d'ammonium et 1 à 2 g/L de phosphate. Selon un mode de réalisation de l'invention, le milieu de culture contient également des macroéléments, des oligoéléments et des vitamines.

Selon un mode de réalisation, la solution nutritive utilisée dans l'étape de maturation contient une source de carbone (par exemple du glucose ou du glycérol), une source d'azote (par exemple du $(NH_4)2SO_4$, une source de phosphate (par exemple du $KH_2PO_4$). De manière préférée, le ratio entre le C (carbone), le N (l'azote) et le P (le phosphate) (C/N/P) est 530 :11 :1 avec toujours la possibilité d'avoir jusqu'à aux fois plus ou moins d'azote (N), et/ou jusqu'à deux fois plus ou moins de phosphate (P), par exemple les ratios suivants peuvent être utilisés 530:22:1,530:5,5:1; 530:22:0,5; 530:11:2; 530:22:2; 530:5,5:0,5; 530:11:0,5; 530:5,5:2.

En général, la culture selon l'étape b) de maturation des souches des levures pour la production de pigments caroténoïdes peut se faire en milieu de culture comprenant environ 10 à 100 g/L de substrat carboné organique, environ 0,15 à 15 g/L d'ammonium et environ 0,9 à 9 g/L de phosphate. A titre d'exemple pour la production d'astaxanthine par *Rhodotorula glutinis,* le milieu de culture comprend 20 g/L glucose, environ 3 g/L d'ammonium et 1 g/L de phosphate.

Selon un mode de réalisation l'étape b) se fait à une température plus bas que l'étape a). Les inventeurs ont constaté que, selon ce mode de réalisation, la quantité de lipide, notamment de DHA produite est augmenté par rapport une culture ou l'étape b) est fait à la même température que l'étape i). En effet, selon le procédé d'invention, la température optimale de croissance est supérieure à la température optimale de production de DHA. De manière générale une l'étape b) peut est réalisé à 1 à 8 degrés moins que la température à laquelle l'étape i) est réaliser. Par exemple l'étape a) de la culture peut être menée à 25°C tandis que l'étape b) peut être mené à 18°C 19°C, 20°C, 21°C, 22°C ou 23°C. Par exemple l'étape a) de la culture peut être menée à 22°C tandis que l'étape b) peut être mené à 18°C 19°C, 20°C, 21°C. Par exemple l'étape a) de la culture peut être menée à 27°C tandis que l'étape b) peut être mené à 20°C, 21°C, 22°C 23°C 24°C, 25°C, ou 26°C.

Une température basse favorise également l'accumulation de pigments.

Plus particulièrement, les conditions de mixotrophie peuvent être effectuées dans des conditions d'éclairement discontinu et/ou variable au cours du temps. Par éclairement discontinu, il faut entendre un éclairement ponctué par des périodes d'obscurité. L'éclairement peut être notamment sous forme de flashs. Un flash, au sens de l'invention, est un éclairement lumineux de courte durée, c'est-à-dire de moins de 30 minutes. La durée peut être de moins de 15 minutes, de préférence de moins de 5 minutes ou plus préférentiellement encore de moins de 1 minute. Selon certains modes de réalisation de l'invention, la durée du flash peut être de moins d'une seconde. Par exemple, le durée du flash peut être 1/10 d'une seconde, ou 2/10 d'une seconde, ou 3/10 d'une seconde, ou 4/10 d'une seconde ou 5/10 d'une seconde, ou 6/10 d'une seconde, ou 7/10 d'une seconde, ou 8/10 d'une seconde, ou 9/10 d'une seconde. L'éclairement lumineux, ou le flash, est généralement d'une durée supérieure à 15 secondes. La durée du flash est généralement comprise entre 5 secondes et 10 minutes, de préférence entre 10 secondes et 2 minutes, plus préférentiellement entre 20 secondes et 1 minute. Ces durées de flash sont appropriées pour des apports de lumière de « basse fréquence ».

Selon ce dernier mode de réalisation, où le régime d'illumination (l'apport de la lumière) est de « basse fréquence », le nombre de flashs est compris entre environ 2 et 3600 par heure. Il peut être, par exemple, compris entre 100 et 3600 flashs par heure. Il peut être également compris entre 120 et 3000, ou entre 400 et 2500, ou entre 600 et 2000, ou entre 800 et 1500 flashs par heure. Il peut être également compris entre 2 et 200, préférentiellement entre 10 et 150, plus préférentiellement entre 15 et 100, et plus préférentiellement encore entre 20 et 50 par heure.

Selon un mode de réalisation, un flash a une durée entre 1/150000 seconde et 1/1000 seconde. Ces durées de flash sont appropriées pour des régimes d'illumination de « haute fréquence » ?c'est-à-dire avec des fréquences de flash de 150kHz à 1kHz respectivement.

Selon ce mode de réalisation, où le régime d'illumination (l'apport de la lumière) est de « haute fréquence », les flashs peuvent avoir lieu entre 3,6 x$10^5$ et 5,4 x $10^9$ fois par heure. Dans ce cas, la variation de la lumière a une fréquence entre 1kHz et 150kHz, c'est-à-dire entre 1000 et 150 000 flashs par seconde. L'apport de lumière selon ce dernier mode de réalisation de l'invention est nommé « haute fréquence ».

Le nombre de flashs par heure est choisi en fonction de l'intensité et de la durée des flashs (voir ci-dessous). En général, l'intensité de la lumière apportée sous forme de flashs est entre 50 et 1000 $\mu$mol. m$^{-2}$. s$^{-1}$, de préférence entre 50 et 500 $\mu$mol. m$^{-2}$. s$^{-1}$, ou 50 et 400 $\mu$mol. m$^{-2}$. s$^{-1}$, et plus préférentiellement entre 150 et 300 $\mu$mol. m$^{-2}$. s$^{-1}$ (1

$\mu$mol. m$^{-2}$. s$^{-1}$ correspond à 1$\mu$E m$^{-2}$. s$^{-1}$ (Einstein), unité souvent utilisée dans la littérature).

Selon un autre mode de l'invention, l'éclairement peut être variable, ce qui signifie que l'éclairement n'est pas interrompu par des phases d'obscurité, mais que l'intensité lumineuse varie au cours du temps. Cette variation de l'intensité de lumière est régulière et peut être périodique ou cyclique. Selon l'invention, on peut aussi procéder à un apport lumineux alliant des phases d'éclairement continues et discontinues.

Par éclairement variable, on entend que l'intensité de la lumière varie de manière régulière au moins deux fois par heure. Un exemple des conditions d'éclairement adaptées à la méthode d'invention est décrit dans la demande de brevet français N° 1057380.

L'éclairement présente, de préférence, des variations d'intensité dont l'amplitude est généralement comprise entre 50 $\mu$mol. m$^{-2}$. s$^{-1}$ et 2000 $\mu$mol. m$^{-2}$. s$^{-1}$, de préférence entre 50 et 1500, plus préférentiellement entre 50 et 200 $\mu$mol. m$^{-2}$. s$^{-1}$.

Selon un mode de réalisation préféré, l'éclairement présente des variations d'intensité dont l'amplitude est comprise entre 50 et 1000 $\mu$mol. m-2.s-1, de préférence entre 50 et 400 $\mu$mol. m-2.s-1, ces variations ayant lieu entre 2 et 3600, de préférence entre 2 et 200 fois par heure.

Ces variations peuvent généralement avoir lieu entre 2 et 3600 fois par heure, de préférence entre 50 et 500 fois, plus préférentiellement entre 100 et 200 fois par heure. Ces conditions de culture permettent d'apporter une quantité définie de lumière. Cet apport lumineux peut comporter des phases d'éclairement discontinu et/ou variable, avec des variations d'intensité pouvant avoir des amplitudes identiques ou différentes.

Il est apparu qu'un éclairement variable et/ou discontinu des cultures, en particulier dans la mise en oeuvre d'une culture en mode mixotrophe, avait un impact favorable sur le développement des cellules ou la production de molécules d'intérêt, en particulier des protistes, et des cellules végétales, et permettait d'accroître la productivité de celles-ci, notamment en ce qui concerne leur production de molécule d'intérêt comme des lipides ou des pigments. Sans être lié par la théorie, l'inventeur estime qu'un apport discontinu et/ou variable de lumière aux cellules a pour effet de provoquer un « stress » favorable à la croissance et à la synthèse des lipides. Ce phénomène pourrait s'expliquer, en partie, par le fait que dans la nature, les cellules ont tendance à accumuler des réserves lipidiques pour résister aux contraintes de leur environnement.

Selon un mode de réalisation de l'invention, dans le cas de la mixotrophie, la culture selon l'étape a) se fait en présence des flashs. L'intensité des flashs est comprise entre 50 et 200 $\mu$mol. m$^{-2}$. s$^{-1}$, la durée des flashs est comprise entre environ 1/10ème de seconde et cinq minutes, de préférence entre environ une seconde et une minute et le nombre de flashs par heure peut varier entre 2 et 3600, de préférence entre 2 et 360 par heure.

Selon un mode de réalisation de l'invention, la culture selon l'étape b) se fait en présence des flashs. L'intensité des flashs est comprise entre 200 et 2000 $\mu$mol. m$^{-2}$. s$^{-1}$, la durée des flashs est comprise entre environ 1/10ème de seconde et dix minutes, de préférence entre environ 1/10ème d'une seconde et cinq minutes et le nombre de flashs par heure peut varier entre 2 et 3600, de préférence entre 20 et 1000 par heure.

Selon un mode de l'invention et quelles que soient les conditions d'éclairement, l'intensité de la lumière apportée à la culture en étape a) et en étape b) varie en fonction de la densité cellulaire. Plus la culture devient dense, plus la lumière peut être intense. La densité cellulaire est le nombre de cellules par mL et est mesurée selon les techniques connues de l'homme du métier.

Par exemple, pour des protistes, des bactéries et des levures, au stade initial de la culture selon l'étape a) quand la densité cellulaire est entre environ 10$^7$ et 5 x10$^7$ cellules par mL, et pour une durée de flashs variant entre 1 seconde à 60 minutes avec 2 et 360 flashs par heure, l'intensité lumineuse peut être entre 50 et 200 $\mu$mol. m$^{-2}$. s$^{-1}$, de préférence entre 50 et 150 $\mu$mol. m$^2$. s$^{-1}$. Quand la culture atteint une densité entre 10$^8$ et 10$^9$ cellules par mL, l'intensité lumineuse peut être augmentée jusqu'à entre 100 et 500 $\mu$mol. m$^{-2}$. s$^{-1}$, par exemple, de préférence, entre 200 et 400 $\mu$mol. m$^{-2}$. s$^{-1}$. Quand la culture, au stade final, atteint une densité entre 10$^9$ et 10$^{10}$ cellules par mL, l'intensité lumineuse peut être augmentée jusqu'à entre 200 et 800 $\mu$mol. m$^{-2}$. s$^{-1}$, par exemple, de préférence, entre 200 et 500 $\mu$mol. m$^{-2}$. s$^{-1}$.

Pour la culture des protistes, des bactéries et des levures selon l'étape b) pour une durée de flashs variant entre 1/10ème d'une seconde à 30 minutes avec 2 et 3600 flashs par heure, l'intensité lumineuse peut être augmentée jusqu'à entre 200 et 2000 $\mu$mol. m$^{-2}$. s$^{-1}$, par exemple, de préférence, entre 300 et 1000 $\mu$mol. m$^{-2}$. s$^{-1}$.

Selon certains modes de réalisation, par exemple, quand la durée des flashs est par exemple moins d'une minute, ou moins d'une seconde, l'intensité de la lumière peut être plus importante par rapport aux valeurs citées ci-dessus.

Comme détaillé plus loin (voir les Tableaux 3A et 3B), les genres de protistes visés par le procédé de l'invention tombent dans deux catégories : le Groupe 1 (« non photo-synthétique ») et le Groupe 2 « photo-synthétique ». De manière générale, en en théorie, les souches du Groupe 1 auront moins besoin, ou pourront exploiter différemment la lumière pour croître et pour produire des molécules d'intérêt par rapport au Groupe 2. Par contre, des souches individuelles pourront avoir des besoins en lumières différents. L'homme de métier sait ajuster les paramètres exacts de lumière selon la souche en culture en fonction de ses besoins.

Selon un mode de réalisation de l'invention, la culture des souches appartenant aux genres *Schizochytrium,*

*Aurantiochytrium* et *Crypthecodinium* (Groupe 1) est réalisée avec une intensité de lumière entre 75 à 1200 $\mu$mol. m$^{-2}$. s$^{-1}$, préférentiellement entre 75 à 800 $\mu$mol. m$^{-2}$. s$^{-1}$, plus préférentiellement entre 150 à 600 $\mu$mol. m$^{-2}$. s$^{-1}$. Selon ce mode de réalisation, l'intensité de la lumière varie entre 2 et 200 fois par heure. L'amplitude des variations d'intensité est comprise entre 70 et 400 $\mu$mol. m$^{-2}$. s$^{-1}$, 70 et 300 $\mu$mol. m$^{-2}$. s$^{-1}$, et de préférence entre 100 et 200 $\mu$mol. m$^{-2}$. s$^{-1}$.

Selon un mode de réalisation de l'invention, la culture des souches appartenant aux genres *Schizochytrium, Aurantiochytrium* et *Crypthecodinium* (Groupe 1) est réalisée avec des flashs.

Par exemple, selon un mode de réalisation, la culture des genres du Groupe 1 est illuminée avec des flashs d'une intensité de 200 $\mu$mol. m$^{-2}$. s$^{-1}$, avec 30 flashs par heure.

Selon un autre mode de réalisation de l'invention, la culture des souches appartenant aux genres *Tetraselmis, Scenedesmus, Chlorella, Nitzschia* et *Haematococcus* (Groupe 2) est réalisée avec une intensité de lumière entre 75 à 800 $\mu$mol. m$^{-2}$. s$^{-1}$, préférentiellement de 75 à 600 $\mu$mol. m$^{-2}$. s$^{-1}$ plus préférentiellement de 75 à 250 $\mu$mol. m$^{-2}$. s$^{-1}$. Selon ce mode de réalisation, l'intensité de la lumière varie entre 2 et 3600 fois par heure. L'amplitude des variations d'intensité est comprise entre 70 et 400 $\mu$mol. m$^{-2}$. s$^{-1}$, 70 et 300 $\mu$mol. m$^{-2}$. s$^{-1}$, et de préférence, entre 100 et 200 $\mu$mol. m$^{-2}$. s$^{-1}$. Selon un mode de réalisation de l'invention, la culture des souches appartenant aux genres *Tetraselmis, Scenedesmus, Chlorella, Nitzschia* et *Haematococcus* (Groupe 2) est réalisée avec des flashs.

Par exemple, selon un mode de réalisation, la culture des genres de *Tetraselmis* et *Scenedesmus* est illuminée avec des flashs d'une intensité de 100 $\mu$mol. m$^{-2}$. s$^{-1}$, avec 120 flashs par heure.

Par exemple, selon un mode de réalisation, la culture de souches du genre *Chlorella* est illuminée avec une lumière variable, l'intensité de la lumière variant entre 50 et 100 $\mu$mol. m$^{-2}$. s$^{-1}$, 30 fois par heure.

Par exemple, selon un mode de réalisation, la culture de souches du genre *Haematococcus* est illuminée avec une lumière variable, l'intensité de lumière variant entre 100 et 150 $\mu$E, 60 fois par heure.

Par exemple, selon un mode de réalisation, la culture de souches du genre *Nitzschia* est illuminée avec des flashes, d'une intensité de 200 $\mu$E, 30 fois par heure.

Comme décrit pour l'intensité lumineuse ci-dessus, et selon un mode de l'invention, la quantité de lumière apportée à la culture par heure peut varier en fonction de la densité cellulaire. Au stade initial de la culture, quand la densité cellulaire est de $10^5$ et $5 \times 10^5$ cellules par ml, l'apport total de lumière dans l'heure est généralement compris entre environ 1500 et 8000, de préférence 1500 et 6000 $\mu$mol. m$^{-2}$, de préférence encore entre 2000 et 5000 $\mu$mol. m$^{-2}$. Quand la culture atteint une densité entre $10^6$ et $10^7$ cellules par ml, l'apport total de lumière dans l'heure peut être augmenté jusqu'à entre 6000 et 67 000 $\mu$mol. m$^{-2}$, de préférence entre 6000 et 50 000, et de préférence encore entre 12 000 et 45 000 $\mu$mol. m$^{-2}$, par exemple. Au stade final de la culture, à une densité cellulaire entre $10^7$ et $10^8$ cellules par ml, l'apport total de lumière dans l'heure peut être augmenté jusqu'à entre 45 000 et 300 000, par exemple, de préférence, entre 45 000 et 200 000 $\mu$mol. m$^{-2}$, et par exemple, de préférence encore, entre 50 000 et 150 000 $\mu$mol. m$^{-2}$.

Au stade final de la culture (à une densité cellulaire entre $10^7$ et $10^8$ cellules par ml), la culture est illuminée avec 30 flashs par heure, chaque flash ayant une durée de 30 secondes et une intensité entre 50 et 150 $\mu$mol. m$^{-2}$. s$^{-1}$, ce qui donne un apport total de lumière par heure de 45 000 à 135 000 $\mu$mol. m$^{-2}$.

Selon un mode de l'invention, par exemple, quand la durée des flashs est par exemple de moins d'une minute, ou moins d'une seconde, au stade initial de la culture (à une densité cellulaire entre $10^5$ et $5 \times 10^5$ cellules par ml), la culture est illuminée avec 30 flashs par heure, chaque flash ayant une durée de 10 secondes et une intensité entre 50 et 100 $\mu$mol. m$^{-2}$. s$^{-1}$, ce qui donne un apport total de lumière par heure de 15 000 $\mu$mol. m$^{-2}$ à 30 000 $\mu$mol. m$^{-2}$. Puis, au stade intermédiaire (à une densité cellulaire entre $10^6$ et $10^7$ cellules par ml), la culture est illuminée avec 50 flashs par heure, chaque flash ayant une durée de 10 secondes et une intensité entre 200 et 300 $\mu$mol. m$^{-2}$. s$^{-1}$, ce qui donne un apport total de lumière par heure de 100 000 à 150 000 $\mu$mol. m$^{-2}$. Puis, au stade final de la culture (à une densité cellulaire entre $10^7$ et $10^8$ cellules par ml), la culture est illuminée avec 120 flashs par heure, chaque flash ayant une durée de 10 secondes et une intensité entre 350 et 450 $\mu$mol. m$^{-2}$. s$^{-1}$, ce qui donne un apport total de lumière par heure de 420 000 à 540 000 $\mu$mol. m$^{-2}$.

L'apport de lumière dans les cultures peut être obtenu par des sources de lumière réparties autour de la paroi externe des fermenteurs. Une horloge déclenche ces lampes pour des temps d'éclairement définis. Selon un mode de réalisation préféré, l'éclairage des cultures de l'étape a) et/ou b) est assuré par un dispositif d'illumination interne au fermenteur. Ainsi, l'efficacité de l'illumination est meilleure par rapport à une configuration où la lumière pénètre par des hublots à partir de sources disposées à l'extérieur. Les dispositifs d'illumination peuvent être disposés sur l'ensemble tournant muni de pales ou sur des pièces tubulaires plongeant dans la masse à traiter, ou encore au fond ou au plafond de la cuve. Selon un mode de réalisation préféré de l'invention, le dispositif d'illumination est situé sur les contre-pales à l'intérieur du fermenteur. Un tel dispositif est décrit dans la demande de brevet français N° 1353641. Le fermenteur est ainsi muni d'une pluralité de sources d'illumination portées par des contre-pales, ces dernières ayant pour fonction d'empêcher la formation d'un vortex au sein de la biomasse sous l'action de l'ensemble tournant de brassage. Ces sources d'illumination sont de préférence encapsulées, partiellement ou complètement dans au moins une partie de ces contre pales, dans une matière compatible avec la biomasse et en une épaisseur permettant de diffuser ladite lumière vers l'intérieur de la cuve.

Une matière particulièrement bien adaptée est le polysulfone qui combine une bonne compatibilité avec les normes alimentaires (y compris les normes américaines de la Food and Drug Administration, ou FDA en abrégé), un bon coefficient de transfert thermique (permettant une évacuation de la chaleur vers la masse de micro-organismes et un caractère semi-transparent permettant une bonne transmission de la lumière, si le matériau a une épaisseur choisie entre 1 mm et 5 cm ; en outre, ce matériau conserve ses propriétés après un éventuel traitement thermique de stérilisation ou de nettoyage avec des détergents ou de l'acide. Si l'on modifie la combinaison de caractéristiques souhaitées, d'autres matériaux peuvent être choisis, par exemple le polyuréthane, le polypropylène, un matériau acrylique ou un polycarbonate.

De manière préférée, les sources d'illumination sont des diodes électro-luminescentes (LED en abrégé); il s'agit de sources de lumière bien maîtrisées, aussi bien dans leur mise en oeuvre que dans leurs applications. De telles sources peuvent avoir des spectres d'émission très divers, puisqu'il y a des LED blanches (simulant la lumière solaire), mais aussi des LED à gamme spectrale réduite (par exemple centrées sur une lumière rouge, bleue ou verte). De telles sources d'illumination génèrent moins de chaleur que des bulbes ou des ampoules ; elles ont en outre des dimensions suffisamment petites pour pouvoir être implantées sur les faces des contre-pales sans induire de surépaisseur nuisant à la fonction principale de ces plaques.

Les sources d'illumination peuvent avoir des régimes de commande (on parle aussi de régimes de contrôle ou de pilotage) très variés.

Le procédé selon l'invention a pour avantage d'augmenter le rendement en biomasse obtenu de la culture. Ce procédé a aussi pour avantage d'accroître l'accumulation en molécules d'intérêt. On peut citer en tant qu'exemples de ces molécules d'intérêt des alcools, dont des alcools primaires, des alcools secondaires, des esters d'alcools et des alcools gras, des acides gras (AG) dont des acides gras à courtes chaînes (AGCC), des acides gras à longues chaînes (AGLC), des acides gras à très longues chaînes (AGTLC), des acides gras saturés, des acides gras insaturés ou polyinsaturés, des acides gras branchés à des fonctions polaires ou apolaires, des acides gras hydroxylés, des esters d'acides gras et des acides gras à chaîne aliphatique *iso,* des lipides dont des glycolipides, des glycéro-glycolipides, des phospholipides ou glycérophospholipides, des lipides à base de longues chaînes, des sphingolipides, des stérides et des cérides, des lipides insaponifiables tels que des terpènes, des stéroïdes (dont des stérols : phytostérols, cholestérols, vitamine D et autres vitamines) et des prostaglandines, des esters, des alcanes, des alcènes, des aldéhydes, des cétones, des acides organiques, des antioxydants, des polysaccharides, des pigments, des acides aminés, des MAAs, des polymères pseudopeptidiques d'acides aminés tels que la cyanophycine, des enzymes ou des vitamines, des antibiotiques, des composés à activité pharmacologique, des toxines, des protéines exogènes ou recombinantes, des composés organiques comme des acides, des molécules utilisables en tant que biocarburants, des terpènes tels que des botryococcènes, ou des produits intermédiaires pouvant être convertis en carburant. On peut également citer des molécules utilisées pour fabriquer des bioplastiques comme l'hydroxubutyrate (PHB).

En particulier, ce procédé a pour avantage d'enrichir les cellules ainsi cultivées en acides gras polyinsaturés, plus particulièrement, en acide éicosapentaénoïque (EPA) et/ou acide docosahexaénoïque (DHA) et/ou en acide arachidonique (ARA), et/ou en acide a-linolénique (ALA), et/ou en acide linoléique, et/ou en acide oléique, et/ou en caroténoïdes, plus particulièrement, en lutéine canthaxanthine, astaxanthine, fucoxanthine, zéaxanthine, échinénone, bêta-carotène et phoenicoxanthine.

Les types cellulaires pouvant être cultivés selon le procédé décrit dans cette invention, de préférence, selon des modes de réalisation comprenant l'étape b) en mode mixotrophie, correspondent à tous les organismes unicellulaires photosensibles, eucaryotes, capables de croître en culture mixotrophe. D'ailleurs, il est souhaitable que la capacité à croître en mixotrophie permette d'atteindre des productivités en biomasse ou en molécules d'intérêt compatibles avec une exploitation industrielle. Par « photosensible » on entend des cellules capables d'induire une activité métabolique en réponse à une illumination naturelle ou artificielle, dans des conditions de mixotrophie. Cette activité métabolique peut être par exemple une activité de photosynthèse ou la synthèse d'un pigment.

Les cellules qui peuvent être mises en culture dans le procédé selon l'invention, de préférence, selon des modes de réalisation comprenant l'étape b) en mode mixotrophie, peuvent être choisies parmi des cellules eucaryotes photosensibles isolées à partir d'un organisme pluricellulaire animal, végétal ou fongique, ou des organismes unicellulaires eucaryotes photosensibles.

En tant que microorganismes eucaryotes, on peut citer, non limitativement, les protistes, les levures, marins et d'eau douce. Ces microorganismes peuvent être photosynthétiques ou non. Parmi les protistes non photosynthétiques, on citera, en particulier, ceux de la classe *Labyrinthulomycete,* notamment du genre *Schizochytrium* ou *Aurantiochytrium,* qui sont cultivés en mixotrophie pour la production simultanée d'acide docosahexaénoïque (DHA) et de pigments caroténoïdes comme l'astaxanthine ou la canthaxanthine. Le protiste *Nitzschia brevirostris* est un microorganisme eucaryote photosynthétique qui est cultivé en mixotrophie pour la production simultanée d'acide éicosapentaénoïque (EPA) et de pigments caroténoïdes. D'autres microorganismes eucaryotes photosensibles comme des levures peuvent également être cultivés à l'aide du procédé selon l'invention, de préférence, selon des modes de réalisation comprenant l'étape a) et/ou b) en mode mixotrophie.

En tant que cellules eucaryotes photosensibles, on peut également utiliser, par exemple, des cellules eucaryotes isolées à partir d'un organisme pluricellulaire et capables de croître en culture mixotrophe. Ce type d'organisme peut être fongique, animal ou végétal. L'apport de lumière durant les phases de croissance et de production permet le maintien de l'activité photosynthétique ou d'un métabolisme photo-activé, comme par exemple l'activation de la voie de biosynthèse d'un pigment induite par la lumière. Parmi les cellules eucaryotes issues d'organismes pluricellulaires, on peut notamment citer les cellules végétales non différenciées issues de Cals qui sont maintenues dans cet état par l'adjonction d'hormones végétales dans le milieu de culture ; ou encore les cellules animales qui ont été traitées par un processus d'immortalisation permettant la culture *in vitro*.

Des protistes mixotrophes d'intérêt pouvant être mis en oeuvre dans le procédé selon l'invention peuvent être, par exemple, sélectionnés parmi les espèces des genres suivant : *Schizochytrium, Thraustochytrium, Odontella, Phaeodactylum,* Nannochloris *Crypthecodinium, Monodus, Nannochloropsis, Isochrysis, Euglena, Cyclotella, Nitzschia, Aurantiochytrium, Scenedesmus* et/ou *Tetraselmis, Chlorella* et *Haematococcus.*

Selon un aspect préféré, les protistes d'intérêt sont choisis parmi les espèces des genres *Schizochytrium, Thraustochytrium, Odontella, Phaeodactylum,* Nannochloris, *Crypthecodinium, Monodus, Nannochloropsis, Isochrysis, Euglena, Cyclotella, Nitzschia, Aurantiochytrium, Scenedesmus,* et notamment parmi les espèces des genres *Schizochytrium, Thraustochytrium, Odontella, Phaeodactylum,* Nannochloris, *Crypthecodinium, Monodus, Nannochloropsis, Chlorella* et *Haematococcus.*

Concernant les caroténoïdes, les protistes suivants sont particulièrement avantageux: *Schizochytrium* (astaxanthine), *Nitzschia* (fucoxanthine), *Aurantiochytrium* (canthaxanthine ou astaxanthine) et *Scenedesmus* (lutéine).

Les **Tableaux 3A et 3B** indiquent des nouvelles souches identifiées par les inventeurs, qui ont été déposées auprès de la CCAP (Culture Collection of Algae and Protozoa, Scottish Association for Marine Science, Dunstaffnage Marine Laboratory, Oban, Argyll PA371QA, Ecosse, Royaume-Uni) le 8 juillet 2014, selon les dispositions du Traité de Budapest, sous les numéros d'accession CCAP indiqués dans ces Tableaux. Les molécules produites par des souches sont également indiquées.

Ces genres forment deux groupes: le Groupe 1 est constitué des souches appartenant aux genres *Schizochytrium, Aurantiochytrium* et *Crypthecodinium.* Ces protistes n'ont pas de chloroplaste et donc sont dits « non-photosynthétiques ». *Schizochytrium* et *Aurantiochytrium* sont des champignons unicellulaires, et *Crypthecodinium* est un flagellé hétérotrophe. Les exemples de ces genres sont donnés dans le **Tableau 3A** ci-dessous et indiqué par Groupe 1. Selon un mode de réalisation, le procédé d'l'invention vise la culture des genres *Schizochytrium, Aurantiochytrium* et *Crypthecodinium.*

Le deuxième groupe, le Groupe 2, est constitué des souches qui sont photo-synthétiques, c'est-à-dire ayant des chloroplastes. Ces genres de protistes *Tetraselmis, Scenedesmus, Chlorella, Nitzschia* et *Haematococcus* sont classés en tant que microalgues. Selon un mode de réalisation, le procédé de l'invention vise la culture des genres *Tetraselmis, Scenedesmus, Chlorella, Nitzschia* et *Haematococcus.*

**Groupe 1**

**Tableau 3A** : Exemples des souches qui peuvent être mises en oeuvre selon le procédé de l'invention. Ces souches sont des protistes dits non photosynthétiques, c'est-à-dire n'ayant pas de chloroplaste. Selon un mode de réalisation, le procédé de l'invention vise la culture appartenant aux genres *Schizochytrium, Aurantiochytrium* et *Crypthecodinium.*

| Souches | N° de Dépôt CCAP | Molécule d'intérêt |
|---|---|---|
| Schizochytrium sp. FCC 36 | CCAP 4087/3 | DHA, astaxanthine |
| Schizochytrium sp. FCC1320 | CCAP 4087/4 | DHA, astaxanthine |
| Schizochytrium sp. FCC1491 | CCAP 4087/5 | DHA, astaxanthine |
| Aurantiochytrium mangrovei FCC 1311 | CCAP 4062/3 | DHA, canthaxanthine, astaxanthine |
| Aurantiochytrium mangrovei FCC 31 | CCAP 4062/2 | DHA, canthaxanthine, astaxanthine |
| Aurantiochytrium mangrovei FCC 1319 | CCAP 4062/4 | DHA, cantha xanthine, astaxanthine |
| Aurantiochytrium mangrovei FCC 1479 | CCAP 4062/6 | DHA, canthaxanthine, astaxanthine |
| Aurantiochytrium mangrovei FCC 1325 | CCAP 4062/5 | DHA, canthaxanthine, astaxanthine |
| Crypthecodinium cohnii FCC 30 | CCAP 1104/3 | DHA, caroténoïdes ($\beta$-carotène) |
| Crypthecodinium cohnii FCC 1384 | CCAP 1104/5 | DHA, caroténoïdes ($\beta$-carotène) |

(suite)

| Souches | N° de Dépôt CCAP | Molécule d'intérêt |
|---|---|---|
| Crypthecodinium cohnii FCC 1348 | CCAP 1104/4 | DHA, caroténoïdes (β-carotène) |

**Groupe 2**

**Tableau 3B** : Exemples des souches qui peuvent être mises en oeuvre selon le procédé de l'invention. Ces souches sont des microalgues dites photosynthétiques, c'est-à-dire ayant des chloroplastes. Ces genres sont des microalgues. Selon un mode de réalisation, le procédé d'l'invention vise la culture des genres *Tetraselmis, Scenedesmus, Chlorella, Nitzschia et Haematococcus.*

| Souches | N° de Dépôt CCAP | Molécule d'intérêt |
|---|---|---|
| Tetraselmis sp. FCC 1563 | CCAP 66/85 | EPA, ALA |
| Scenedesmus abundans FCC 23 | CCAP 276/78 | ALA, acide oléique, lutéine |
| Scenedesmus sp. FCC 1483 | CCAP 276/79 | ALA, acide oléique, lutéine |
| Scenedesmus obliquus FCC 4 | CCAP 276/77 | ALA, acide oléique, lutéine |
| Chlorella sorokiniana FCC 2 | CCAP 211/129 | lutéine |
| Chlorella sp. FCC 1553 | CCAP 211/130 | lutéine |
| Chlorella sp. FCC 1520 | CCAP 211/131 | lutéine |
| Nitzschia sp. FCC 1687 | CCAP 1052/22 | EPA, fucoxanthine |
| Haematococcus sp. FCC 1643 | CCAP 34/18 | astaxanthine |

Sont décrits également les nouvelles souches des **Tableaux 3A et 3B.** Ces souches ont été sélectionnées pour leur caractère mixotrophe et pour leur haut rendement en acides gras et/ou en caroténoïdes, notamment en lutéine, fucoxanthine, astaxanthine, canthaxanthine et β-carotène, et notamment pour leur capacité à être cultivées avec un apport lumineux supérieur à 10 μE, dans un milieu riche en éléments organiques. Ces milieux sont connus de l'homme du métier.

Le procédé de culture selon l'invention peut s'appliquer également à toute espèce du genre *Schizochytrium, Aurantiochytrium, Crypthecodinium, Tetraselmis, Scenedesmus, Chlorella, Nitzschia* et *Haematococcus* capable de croître dans les conditions mixotrophes selon l'invention, et capable de produire des acides gras et/ou des caroténoïdes.

Lorsque les microalgues sont du genre Chlorella, elles pourront être choisies parmi les espèces *C. acuminata, C. angustoellipsoidea, C. anitrata, C. antarctica, C. aureoviridis, C. autotrophica, C. botryoides, C. caldaria, C. candida, C. capsulata, C. chlorelloides, C. cladoniae, C. coelastroides, C. colonialis, C. communis, C. conductrix, C. conglomerata, C. desiccata, C. ellipsoidea, C. elongata, C. emersonii, C. faginea, C. fusca, C. glucotropha, C. homosphaera, C. infusionum, C. kessleri, C. koettlitzii, C. lacustris, C. lewinii, C. lichina, C. lobophora, C. luteo-viridis, C. marina, C. miniata, C. minor, C. minutissima, C. mirabilis, C. mucosa, C. mutabilis, C. nocturna, C. nordstedtii, C. oblonga, C. oocystoides, C. ovalis, C. paramecii, C. parasitica, C. parva, C. peruviana, C. photophila, C. pituita, C. pringsheimii, C. protothecoides, C. pulchelloides, C. pyrenoidosa, C. regularis, C. reisiglii, C. reniformis, C. rotunda, C. rubescens, C. rugosa, C. saccharophila, C. salina, C. simplex, C. singularis, C. sorokiniana, C. spaerckii, C. sphaerica, C. stigmatophora, C. subsphaerica, C. terricola, C. trebouxioides, C. vannielii, C. variabilis, C. viscosa, C. volutis, C. vulgaris, C. zopfingiensis.* Avantageusement selon l'invention les algues du genre Chlorella pourront être des algues choisies parmi les espèces *C. sorokiniana ou C. vulgaris.*

Lorsque les microalgues sont du genre Scenedesmus, elles pourront être choisies parmi les espèces *S. abundans, S. aciculatus, S. aculeolatus, S. aculeotatus, S. acuminatus, S. acutiformis, S. acutus, S. aldavei, S. alternans, S. ambuehlii, S. anhuiensis, S. anomalus, S. antennatus, S. antillarum, S. apicaudatus, S. apiculatus, S. arcuatus, S. aristatus, S. armatus, S. arthrodesmiformis, S. arvernensis, S. asymmetricus, S. bacillaris, S. baculiformis, S. bajacalifornicus, S. balatonicus, S. basiliensis, S. bernardii, S. bicaudatus, S. bicellularis, S. bidentatus, S. bijuga, S. bijugatus, S. bijugus, S. brasiliensis, S. breviaculeatus, S. brevispina, S. caribeanus, S. carinatus, S. caudato-aculeolatus, S. caudatus, S. chlorelloides, S. circumfusus, S. coalitus, S. costatogranulatus, S. crassidentatus, S. curvatus, S. decorus, S. denticulatus, S. deserticola, S. diagonalis, S. dileticus, S. dimorphus, S. disciformis, S. dispar, S. distentus, S. ecornis,*

*S. ellipsoideus, S. ellipticus, S. falcatus, S. fenestratus, S. flavescens, S. flexuosus, S. furcosus, S. fuscus, S. fusiformis, S. gracilis, S. graevenitzii, S. grahneisii, S. granulatus, S. gujaratensis, S. gutwinskii, S. hanleyi, S. helveticus, S. heteracanthus, S. hindakii, S. hirsutus, S. hortobagyi, S. houlensis, S. huangshanensis, S. hystrix, S. incrassatulus, S. indianensis, S. indicus, S. inermis, S. insignis, S. intermedius, S. javanensis, S. jovais, S. jugalis, S. kerguelensis, S. kissii, S. komarekii, S. lefevrei, S. linearis, S. littoralis, S. longispina, S. longus, S. luna, S. lunatus, S. magnus, S. maximus, S. microspina, S. minutus, S. mirus, S. morzinensis, S. multicauda, S. multiformis, S. multispina, S. multistriatus, S. naegelii, S. nanus, S. notatus, S. nygaardii, S. oahuensis, S. obliquus, S. obtusiusculus, S. obtusus, S. olvalternus, S. oocystiformis, S. opoliensis, S. ornatus, S. ovalternus, S. pannonicus, S. papillosum, S. parisiensis, S. parvus, S. pecsensis, S. pectinatus, S. perforatus, S. planctonicus, S. plarydiscus, S. platydiscus, S. pleiomorphus, S. polessicus, S. polydenticulatus, S. polyglobulus, S. polyspinosus, S. praetervisus, S. prismaticus, S. producto-capitatus, S. protuberans, S. pseudoarmatus, S. pseudobernardii, S. pseudodenticulatus, S. pseudogranulatus, S. pseudohystrix, S. pyrus, S. quadrialatus, S. quadricauda, S. quadricaudata, S. quadricaudus, S. quadrispina, S. raciborskii, S. ralfsii, S. reginae, S. regularis, S. reniformis, S. rostrato-spinosus, S. rotundus, S. rubescens, S. scenedesmoides, S. schnepfii, S. schroeteri, S. securiformis, S. semicristatus, S. semipulcher, S. sempervirens, S. senilis, S. serrato-perforatus, S. serratus, S. serrulatus, S. setiferus, S. sihensis, S. smithii, S. soli, S. sooi, S. spicatus, S. spinoso-aculeolatus, S. spinosus, S. spinulatus, S. striatus., S. subspicatus, S. tenuispina, S. terrestris, S. tetradesmiformis, S. transilvanicus, S. tricostatus, S. tropicus, S. tschudyi, S. vacuolatus, S. variabilis, S. velitaris, S. verrucosus, S. vesiculosus, S. westii, S. weberi, S. wisconsinensis, S. wuhanensis, S. wuhuensis.* Avantageusement selon l'invention les algues du genre Scenedesmus pourront être des algues choisies parmi les espèces *S. obliquus* ou *S. abundans.*

Lorsque les microalgues sont du genre Nitzschia, *N. abbreviata, N. abonuensis, N. abridia, N. accedens, N. accommodata, N. aciculariformis, N. acicularioides, N. acicularis* (comprenant toutes ces variétés), *N. acidoclinata, N. actinastroides, N. actydrophila, N. acula, N. acuminata* (comprenant toutes ces variétés), *N. acuta, N. adamata, N. adamatoides, N. adapta, N. adducta, N. adductoides, N. admissa, N. admissoides, N. aequalis, N. aequatorialis, N. aequora, N. aequorea, N. aerophila, N. aerophiloides, N. aestuari, N. affinis, N. africana, N. agnewii, N. agnita, N. alba, N. albicostalis, N. alexandrina, N. alicae, N. allanssonii, N. alpina, N. alpinobacillum, N. amabilis, N. ambigua, N. americana, N. amisaensis, N. amphibia, N. amphibia* (comprenant toutes ces variétés), *N. amphibioides, N. amphicephala, N. amphilepta, N. amphioxoides, N. amphioxys* (comprenant toutes ces variétés), *N. amphiplectans, N. amphiprora, N. amplectens, N. amundonii, N. anassae, N. andicola, N. angularis* (comprenant toutes ces variétés), *N. angulata, N. angustata* (comprenant toutes ces variétés), *N. angustatula, N. angustiforaminata, N. aniae, N. antarctica, N. antillarum, N. apiceconica, N. apiculata, N. archibaldii, N. arcuata, N. arcula, N. arcus, N. ardua, N. aremonica, N. arenosa, N. areolata, N. armoricana, N. asperula, N. astridiae, N. atomus, N. attenuata, N. aurantiaca, N. aurariae, N. aurica, N. auricula, N. australis, N. austriaca, N. bacata* (comprenant toutes ces variétés), *N. bacillariaeformis, N. bacilliformis, N. bacillum, N. balatonis, N. balcanica, N. baltica, N. barbieri* (comprenant toutes ces variétés), *N. barkleyi, N. barronii, N. barrowiana, N. bartholomei, N. bathurstensis, N. bavarica, N. behrei, N. bergii, N. beyeri, N. biacrula, N. bicapitata* (comprenant toutes ces variétés), *. bicuneata, N. bifurcata, N. bilobata* (comprenant toutes ces variétés), *N. birostrata, N. bisculpta, N. bita, N. bizertensis, N. blankaartensis, N. bombiformis, N. borealis, N. bosumtwiensis, N. braarudii, N. brebissonii* (comprenant toutes ces variétés), *N. bremensis* (comprenant toutes ces variétés), *N. brevior, N. brevirostris, N. brevissima* (comprenant toutes ces variétés), *N. brevistriata, N. brightwellii, N. brittonii, N. brunoi, N. bryophila, N. buceros, N. bukensis, N. bulnheimiana, N. buschbeckii, N. calcicola, N. caledonensis, N. calida* (comprenant toutes ces variétés), N. *californica, N. campechiana, N. capensis, N. capitata, N. capitellata* (comprenant toutes ces variétés), *N. capuluspalae, N. carnicobarica, N. carnico-barica, N. challengeri, N. chalonii, N. chandolensis, N. chardezii, N. chasei, N. chauhanii, N. chungara, N. chutteri, N. circumsuta, N. clarissima, N. clausii, N. clementei, N. clementia, N. clevei, N. closterium* (comprenant toutes ces variétés), *N. coarctata, N. cocconeiformis, N. communis* (comprenant toutes ces variétés), *N. commutata, N. commutatoides, N. compacta, N. compressa* (comprenant toutes ces variétés), *N. concordia, N. confinis, N. conformata, N. confusa, N. congolensis, N. constricta* (comprenant toutes ces variétés), N. *consummata, N. corpulenta, N. costei, N. coutei, N. creticola, N. cucumis, N. cursoria, N. curta, N. curvata, N. curvilineata, N. curvipunctata, N. curvirostris* (comprenant toutes ces variétés), *N. curvula* (comprenant toutes ces variétés), *N. cuspidata, N. cylindriformis, N. cylindrus, N. dakariensis, N. davidsonii, N. dealpina, N. debilis, N. decipiens, N. delauneyi, N. delicatissima, N. delicatula, N. delognei, N. denticula* (comprenant toutes ces variétés), *N. denticuloides, N. desertorum, N. dianae, N. diaphana, N. diducta, N. didyma, N. dietrichii, N. dilatata, N. diluviana, N. dippelii, N. directa, N. diserta, N. disputata, N. dissipata* (comprenant toutes ces variétés), *N. dissipatoides, N. distans* (comprenant toutes ces variétés), *N. distantoides, N. divaricata, N. divergens, N. diversa, N. diversecostata, N. doljensis, N. draveillensis, N. droebakensis, N. dubia* (comprenant toutes ces variétés), *N. dubiformis, N. dubioides, N. ebroicensis, N. eglei, N. elegans, N. elegantula, N. elegens, N. elliptica,* **N. elongata,** *N. entomon, N. epiphytica, N. epiphyticoides, N. epithemiformis, N. epithemioides, N. epithemoides* (comprenant toutes ces variétés), *N. epsilon, N. erlandssonii, N. erosa, N. etoshensis, N. examinanda, N. eximia, N. famelica, N. fasciculata, N. febigeri, N. ferox, N. ferrazae, N. fibula-fissa, N. filiformis* (comprenant toutes ces variétés), *N. flexa, N. flexoides, N. fluminensis, N. fluorescens, N. fluvialis, N. fogedii, N. fonticola* (comprenant toutes ces variétés), *N. fonticoloides, N. fonticula, N. fontifuga, N. forfica, N. formosa, N. fossalis, N. fossilis, N. fragilariiformis,*

*N. franconica, N. fraudulenta, N. frauenfeldii, N. frequens, N. frickei, N. frigida* (comprenant toutes ces variétés), *N. frustuloides, N. frustulum* (comprenant toutes ces variétés), *N. fruticosa, N. fundi, N. fusiformis, N. gaarderi, N. gaertnerae, N. gandersheimiensis, N. garrensis, N. gazellae, N. geitleri, N. geitlerii, N. gelida* (comprenant toutes ces variétés), *N. geniculata, N. gessneri, N. gieskesii, N. gigantea, N. gisela, N. glabra, N. glacialis* (comprenant toutes ces variétés), *N. glandiformis, N. goetzeana* (comprenant toutes ces variétés), *N. gotlandica, N. graciliformis, N. gracilis* (comprenant toutes ces variétés), *N. gracillima, N. graciloides, N. gradifera, N. graeffii, N. grana, N. grandis, N. granii* (comprenant toutes ces variétés), *N. granulata* (comprenant toutes ces variétés), *N. granulosa, N. groenlandica, N. grossestriata, N. grovei, N. gruendleri, N. grunowii, N. guadalupensis, N. guineensis, N. guttula, N. gyrosigma, N. habirshawii, N. habishawii, N. hadriatica, N. halteriformis, N. hamburgiensis, N. hantzschiana* (comprenant toutes ces variétés), *N. harderi, N. harrissonii, N. hassiaca, N. heidenii, N. heimii, N. hemistriata, N. heteropolica, N. heuflerania, N. heufleriana* (comprenant toutes ces variétés), *N. hiemalis, N. hiengheneana, N. hierosolymitana, N. hoehnkii, N. holastica, N. hollerupensis, N. holsatica, N. homburgiensis, N. hudsonii, N. hummii, N. hungarica* (comprenant toutes ces variétés), *N. hustedti, N. hustedtiana, N. hyalina, N. hybrida* (comprenant toutes ces variétés), *N. hybridaeformis, N. ignorata* (comprenant toutes ces variétés), *N. iltisii, N. impressa, N. improvisa, N. incerta, N. incognita, N. inconspicua, N. incrustans, N. incurva* (comprenant toutes ces variétés), *N. indica, N. indistincta, N. inducta, N. inflatula, N. ingenua, N. inimasta, N. innominata, N. insecta, N. insignis* (comprenant toutes ces variétés), *N. intermedia* (comprenant toutes ces variétés), *N. intermissa, N. interrupta, N. interruptestriata, N. invicta* (comprenant toutes ces variétés), *N. invisa, N. invisitata, N. iranica, N. irregularis, N. irremissa, N. irrepta, N. irresoluta, N. irritans, N. italica, N. janischii, N. jelineckii, N. johnmartinii, N. juba, N. jucunda, N. jugata* (comprenant toutes ces variétés), *N. jugiformis, N. kahlii, N. kanakarum, N. kanayae, N. kavirondoensis, N. kerguelensis, N. kimberliensis, N. kittlii, N. kittonii, N. knysnensis, N. kolaczeckii, N. kotschyi, N. kowiensis, N. krachiensis, N. krenicola, N. kuetzingiana* (comprenant toutes ces variétés), *N. kuetzingii, N. kuetzingioides, N. kurzeana, N. kurzii, N. kützingiana* (comprenant toutes ces variétés), *N. labella, N. labuensis, N. lacrima, N. lacunarum, N. lacunicola, N. lacus-karluki, N. lacustris, N. lacuum, N. laevis, N. laevissima, N. lagunae, N. lagunensis, N. lamprocampa* (comprenant toutes ces variétés), N. *lanceola* (comprenant toutes ces variétés), *N. lanceolata* (comprenant toutes ces variétés), *N. lancettula, N. lancettuloides, N. lange-bertalotii, N. latens, N. latestriata, N. latiuscula, N. lauenbergiana, N. lauenburgiana, N. lecointei, N. leehyi, N. legleri, N. lehyi, N. leistikowii, N. lesbia, N. lesinensis, N. lesothensis, N. leucosigma, N. levidensis* (comprenant toutes ces variétés), *N. liebetruthii* (comprenant toutes ces variétés), *N. ligowskii, N. limicola, N. limulus, N. linearis* (comprenant toutes ces variétés), *N. lineata, N. lineola, N. linkei, N. lionella, N. littoralis* (comprenant toutes ces variétés), *N. littorea, N. longa, N. longicollum, N. longirostris, N. longissima* (comprenant toutes ces variétés), *N. lorenziana* (comprenant toutes ces variétés), *N. lucisensibilis, N. lunaris, N. lunata, N. lurida, N. luzonensis, N. macaronesica, N. macedonica, N. macéra, N. machardyae, N. macilenta* (comprenant toutes ces variétés), *N. magnacarina, N. mahihaensis, N. mahoodii, N. maillardii, N. major, N. majuscula* (comprenant toutes ces variétés), *N. makarovae, N. manca, N. mancoides, N. manguini, N. marginata, N. marginulata* (comprenant toutes ces variétés), *N. marina, N. martiana, N. maxima, N. media, N. medioconstricta, N. mediocris, N. mediterranea, N. metzeltinii, N. microcephala* (comprenant toutes ces variétés), *N. migrans, N. minuta, N. minutissima, N. minutula, N. miramarensis, N. miserabilis, N. mitchelliana, N. modesta, N. moissacensis* (comprenant toutes ces variétés), *N. mollis, N. monachorum, N. monoensis, N. montanestris, N. morosa, N. multistriata, N. nana, N. natalensis, N. natans, N. nathorsti, N. navicularis, N. navis-varingica, N. navrongensis, N. neglecta, N. nelsonii, N. neocaledonica, N. neoconstricta, N. neofrigida, N. neogena, N. neotropica, N. nereidis, N. nicobarica, N. nienhuisii, N. normannii, N. notabilis, N. nova, N. novae-guineaensis, N. novae-guineensis, N. novaehollandiae, N. nova-zealandia, N. nyassensis, N. oberheimiana, N. obesa, N. obliquecostata, N. obscura, N. obscurepunctata, N. obsidialis, N. obsoleta, N. obsoletiformis, N. obtusa* (comprenant toutes ces variétés), *N. obtusangula, N. oceanica, N. ocellata, N. oliffi, N. omega, N. osmophila, N. ossiformis, N. ostenfeldii, N. ovalis, N. paaschei, N. pacifica, N. palacea, N. palea* (comprenant toutes ces variétés), *N. paleacea, N. paleaeformis, N. paleoides, N. palustris, N. pamirensis, N. panduriformis* (comprenant toutes ces variétés), *N. pantocsekii, N. paradoxa* (comprenant toutes ces variétés), *N. parallela, N. pararostrata, N. partita, N. parvula* (comprenant toutes ces variétés), *N. parvuloides, N. paxillifer, N. peisonis, N. pelagica, N. pellucida, N. pennata, N. peragallii, N. perindistincta, N. perminuta, N. perpusilla* (comprenant toutes ces variétés), *N. perspicua, N. persuadens, N. pertica, N. perversa, N. petitiana, N. philippinarum, N. pilum, N. pinguescens, N. piscinarum, N. plana* (comprenant toutes ces variétés), *N. planctonica, N. plicatula, N. plioveterana, N. polaris, N. polymorpha, N. ponciensis, N. praecurta, N. praefossilis, N. praereinholdii, N. princeps, N. procera, N. prolongata* (comprenant toutes ces variétés), *N. prolongatoides, N. promare, N. propinqua, N. pseudepiphytica, N. pseudoamphioxoides, N. pseudoamphioxys, N. pseudoamphyoxys, N. pseudoatomus, N. pseudobacata, N. pseudocapitata, N. pseudocarinata, N. pseudocommunis, N. pseudocylindrica, N. pseudodelicatissima, N. pseudofonticola, N. pseudohungarica, N. pseudohybrida, N. pseudonana, N. pseudoseriata, N. pseudosigma, N. pseudosinuata, N. pseudostagnorum, N. pubens, N. pulcherrima, N. pumila, N. punctata (comprenant toutes ces variétés), N. pungens* (comprenant toutes ces variétés), *N. pungiformis, N. pura, N. puriformis, N. pusilla* (comprenant toutes ces variétés), *N. putrida, N. quadrangula, N. quickiana, N. rabenhorstii, N. radicula* (comprenant toutes ces variétés), *N. rautenbachiae, N. recta* (comprenant toutes ces variétés), N. *rectiformis, N. rectilonga, N. rectirobusta, N. rectissima, N. regula, N. reimeri, N. reimerii, N. reimersenii, N. retusa, N. reversa, N. rhombica, N. rhombiformis, N. rhopalodioides,*

*N. richterae, N. rigida* (comprenant toutes ces variétés), *N. ritscheri, N. robusta, N. rochensis, N. rolandii, N. romana, N. romanoides, N. romanowiana, N. rorida, N. rosenstockii, N. rostellata, N. rostrata, N. ruda, N. rugosa, N. rupestris, N. rusingae, N. ruttneri, N. salinarum, N. salinicola, N. salpaespinosae, N. salvadoriana, N. sansimoni, N. sarcophagum, N. scabra, N. scalaris, N. scaligera, N. scalpelliformis, N. schoenfeldii, N. schwabei, N. schweikertii, N. scutellum, N. sellingii, N. semicostata, N. semirobusta, N. separanda, N. seriata* (comprenant toutes ces variétés), *N. serpenticola, N. serpentiraphe, N. serrata, N. sibula* (comprenant toutes ces variétés), *N. sigma* (comprenant toutes ces variétés), *N. sigmaformis, N. sigmatella, N. sigmoidea* (comprenant toutes ces variétés), *N. silica, N. silicula* (comprenant toutes ces variétés), *N. siliqua, N. similis, N. simplex, N. simpliciformis, N. sinensis, N. sinuata* (comprenant toutes ces variétés), *N. smithii, N. sociabilis, N. socialis* (comprenant toutes ces variétés), *N. solgensis, N. solida, N. solita, N. soratensis, N.* sp., *N. spathulata* (comprenant toutes ces variétés), *N. speciosa, N. spectabilis* (comprenant toutes ces variétés), *N. sphaerophora, N. spiculoides, N. spiculum, N. spinarum, N. spinifera, N. stagnorum, N. steenbergensis, N. stellata, N. steynii, N. stimulus, N. stoliczkiana, N. stompsii* (comprenant toutes ces variétés), *N. strelnikovae, N. stricta, N. strigillata, N. striolata, N. subaccommodata, N. subacicularis, N. subacuta, N. subamphioxioides, N. subapiculata, N. subbacata, N. subcapitata, N. subcapitellata, N. subcohaerens* (comprenant toutes ces variétés), *N. subcommunis, N. subconstricta, N. subcurvata, N. subdenticula, N. subfalcata, N. subfraudulenta, N. subfrequens, N. subfrustulum, N. subgraciloides, N. subinflata, N. subinvicta, N. sublaevis, N. sublanceolata, N. sublica, N. sublinearis, N. sublongirostris, N. submarina, N. submediocris, N. subodiosa, N. subpacifica, N. subpunctata, N. subromana, N. subrostrata, N. subrostratoides, N. subrostroides, N. subsalsa, N. subtilioides, N. subtilis* (comprenant toutes ces variétés), *N. subtubicola, N. subvitrea, N. suchlandtii, N. sulcata, N. sundaensis, N. supralitorea, N. tabellaria, N. taenia, N. taeniiformis, N. tantata, N. tarda, N. taylorii, N. temperei, N. tenella, N. tenerifa, N. tenuiarcuata, N. tenuirostris, N. tenuis* (comprenant toutes ces variétés), *N. tenuissima, N. tergestina, N. terrestris, N. terricola, N. thermalis* (comprenant toutes ces variétés), *N. thermaloides, N. tibetana, N. tirstrupensis, N. tonoensis, N. towutensis, N. translucida, N. tropica, N. tryblionella* (comprenant toutes ces variétés), *N. tsarenkoi, N. tubicola, N. tumida, N. turgidula, N. turgiduloides, N. umaoiensis, N. umbilicata, N. umbonata, N. vacillata, N. vacua, N. valdecostata, N. valdestriata, N. valens, N. valga, N. valida* (comprenant toutes ces variétés), *N. vanheurckii, N. vanoyei, N. vasta, N. ventricosa, N. vermicularioides, N. vermicularis* (comprenant toutes ces variétés), *N. vermicularoides, N. vexans, N. victoriae, N. vidovichii, N. vildaryana, N. villarealii, N. virgata, N. visurgis, N. vitrea (comprenant toutes ces variétés), N. vivax* (comprenant toutes ces variétés), *N. vixnegligenda, N. vonhauseniae, N. vulga, N. weaveri, N. weissflogii, N. westii, N. williamsiii, N. wipplingeri, N. witkowskii, N. wodensis, N. woltereckii, N. woltereckoides, N. wuellerstorfii, N. wunsamiae, N. yunchengensis, N. zebuana, N. zululandica.*

Avantageusement selon l'invention, les algues du genre *Nitzschia* pourront être des algues choisies parmi les espèces *N. sp.*

Lorsque les microalgues sont du genre *Haematococcus,* elles pourront être choisies parmi les espèces *H. allmanii, H. buetschlii, H. capensis, H. carocellus, H. droebakensis, H. grevilei, H. insignis, H. lacustris, H. murorum, H. pluvialis, H. salinus, H, sanguineis, H. thermalis, H. zimbabwiensis.*

Lorsque les microalgues sont du genre *Aurantiochytrium,* elles pourront être choisies parmi les espèces: *A. limacinum, A. mangrovei*

Lorsque les microalgues sont du genre *Schizochytrium,* elles pourront être choisies parmi les espèces: *S. aggregatum, S. limacinum, S. mangrovei, S. minutum, S. octosporum.*

Lorsque les microalgues sont du genre *Crypthecodinium,* elles pourront être choisies parmi les espèces: *C. cohnii, C. setense.*

Lorsque les microalgues sont du genre *Tetraselmis,* elles pourront être choisies parmi les espèces: *T. alacris, T. apiculata, T. arnoldii, T. ascus, T. astigmatica, T. bichlora, T. bilobata, T. bolosiana, T. chui, T. contracta, T. convolutae, T. cordiformis, T. desikacharyi, T. elliptica, T. fontiana, T. gracilis, T. hazenii, T. helgolandica, T. impellucida, T. incisa, T. inconspicua, T. indica, T. levis, T. maculata, T. marina, T. mediterranea, T. micropapillata, T. rubens, T. striata, T. subcordiformis, T. suecica, T. tetrabrachia, T. tetrathele, T. verrucosa, T. viridis, T. wettsteinii.*

La culture de champignons filamenteux peut être réalisée dans des fermenteurs aérés et à agitation mécanique tels que ceux décrits dans l'invention. Toutefois, la culture se fait dans des conditions d'agitation adaptées et connues de l'homme du métier, limitant les effets de cisaillement et permettant la culture en mixotrophie des cellules sous forme de filaments ou de cellules isolées. Les effets de la lumière sur le métabolisme de ces organismes sont connus et des métabolites d'intérêt industriel comme des pigments peuvent être produits lors de culture en mixotrophie [Folia Microbiol (Praha). 2013 Apr 2. Light regulation on growth, development, and secondary metabolism of marine-derived filamentous fungi. Cai M, Fang Z, Niu C, Zhou X, Zhang Y.]

Les genres de champignons qui peuvent être cultivés sont à titre d'exemple *Mortierella alpina* pour la production d'ARA ou *Aspergillus niger* pour la production d'acide citrique.

Le protiste *Aurantiochytrium* peut être cultivé selon le procédé de l'invention pour la production de DHA. De préférence, la culture est réalisée selon un mode de réalisation préféré de l'invention, dans lequel les étapes a) et b) se font en conditions de mixotrophie. A titre d'exemple, la culture de la souche *Aurantiochytrium Mangrovei* FCC 1324, une souche isolée par les présents inventeurs et déposée auprès de la CCAP (Culture Collection of Algae and Protozoa, Scottish Association for Marine Science, Dunstaffnage Marine Laboratory, Oban, Argyll PA371QA, Ecosse, Royaume-Uni) selon les dispositions du Traité de Budapest, sous le numéro d'accession CCAP 4062/1, le 21 juin 2013, selon le procédé de l'invention peut permettre de produire une biomasse riche en DHA et en astaxanthine et/ou canthaxanthine. Ledit DHA peut représenter plus de 40 %, ou plus de 50 %, ou plus de 60 % des lipides totaux contenus dans le protiste, les caroténoïdes étant riches en astaxanthine et/ou canthaxanthine, et ladite astaxanthine et/ou canthaxanthine peut représenter plus de 0,1 %, ou plus de 0,15 %, ou plus de 0,2 % en poids sur le poids total de matière sèche. La souche peut atteindre un niveau de productivité (quantité de produit d'intérêt produit, par litre de culture, par heure) de 0,015 mg/L/h, ou plus de 0,020mg/L/h, ou plus de 0,025 mg/L/h (voir le Tableau 2 dans l'Exemple 1).

La culture de cellules végétales peut être envisagée selon un mode de réalisation de l'invention comme, par exemple, des suspensions cellulaires de la plante renonculacée *Adonis annua.* Cultivée en mixotrophie, *Adonis annua* est capable de produire de l'astaxanthine et de l'adonirubine (métabolite intermédiaire de la biosynthèse de l'astaxanthine à partir du bêta-carotène), qui présente une activité anti-oxydante et anti-tumorale chez l'homme.

La levure *Rhodotorula glutinis,* présente un intérêt industriel de par sa vitesse de croissance, sa richesse en huile et la synthèse d'un pigment caroténoïde, le bêta-carotène. La culture de *Rhodotorula glutinis* peut être envisagée puisque l'illumination a été montrée comme bénéfique pour la croissance [Yen HW, Zhang Z.; Bioresource Technology. 2011 Oct; 102(19):9279-81] et également pour la synthèse de pigments [Bhosale P, Gadre RV.; Lett Appl Microbiol. 2002;34(5):349-53]. La culture découplée permet d'appliquer des paramètres physico-chimiques et/ou d'illumination qui sont orientés vers la croissance des cellules, puis dans une seconde phase, vers l'accumulation d'huile et/ou du pigment d'intérêt.

Quelques avantages de l'invention sont illustrés dans le Tableau 5. Le Tableau 5 illustre les résultats d'une comparaison pour la production de biomasse *d'Aurantiochytrium,* puis l'extraction d'huile riche en acides gras polyinsaturés selon le procédé de l'invention et le procédé discontinu, semi-continu et continu. Le procédé de l'invention (découplé) permet de combiner les avantages du mode continu et semi-continu (« Fed-Batch ») en limitant les phases d'arrêt de production pour le nettoyage et la stérilisation d'équipement, tout en permettant l'obtention d'une biomasse de composition optimale grâce à la phase de maturation.

Avantageusement, le procédé selon le mode de réalisation de l'invention permet, pour la culture des protistes, ou levures, la production d'une biomasse comprenant de 40 g/L à 250 g/L de matière sèche, et préférentiellement au-delà de 80 g/L. Par exemple, *Aurantiochytrium* peut produire 150 g/L comme certaines bactéries ou levures.

Ladite biomasse de protiste peut également comprendre une teneur en matière grasse d'au moins 10 %, de préférence au moins 20 %, plus préférentiellement au moins 30 %. Par exemple, pour *Aurantiochytrium,* la teneur en matière grasse de la biomasse est d'au moins 30 %.

Par exemple, les souches de genre *Schizochytrium* sp, en conditions de mixotrophie selon un mode de réalisation de l'invention, produisent du DHA et de l'astaxanthine. Elles donnent généralement un rendement en biomasse compris entre 80 et 200 g/L, avec un taux de lipides entre 30 et 60% de la matière sèche, dont l'acide docosahexaénoïque (DHA) représente 40 à 60%. Le rendement pour l'astaxanthine est généralement de 0,01 à 0,2% de la matière sèche. On peut citer la souche FCC 36 comme exemple de ces souches

Les souches du genre *Aurantiochytrium,* en conditions de mixotrophie selon un mode de réalisation de l'invention, produisent de DHA et de l'astaxanthine et/ou de la canthaxanthine. Les souches donnent généralement un rendement en biomasse compris entre 80 et 200 g/L, avec un taux de lipides d'environ 50% en matière sèche. L'acide docosahexaénoïque (DHA) représente généralement entre 15 à 50% des acides gras; l'astaxanthine et/ou la canthaxanthine représente(nt) généralement 0,01 à 0,2% de la matière sèche. On peut citer comme exemple de ces souches les souches *d'Aurantiochytrium mangrovei* FCC 1311, FCC 1319, FCC 1325 et FCC 31.

Les souches du genre *Crypthecodinium,* notamment de l'espèce *Crypthecodinium cohnii,* en conditions de mixotrophie selon un mode de réalisation de l'invention, produisent du DHA et des caroténoïdes, plus particulièrement du β-carotène. Les souches donnent généralement un rendement en biomasse compris entre 50 et 200 g/L, avec un taux de lipides entre 10 et 30% en matière sèche. L'acide docosahexaénoïque (DHA) représente généralement entre 15 à 50% des acides gras et les caroténoïdes, plus particulièrement le β-carotène, représentent entre 0,01 à 0,2% de la matière sèche. On peut citer comme exemples de ces souches les souches *Crypthecodinium cohnii* FCC 1384, FCC 1348 et FCC 30.

Les souches du genre *Chlorella,* notamment *Chlorella* sp. et *Chlorella sorokiniana* produisent de la lutéine. Les souches permettent de produire, en conditions de mixotrophie selon un mode de réalisation de l'invention, généralement entre 60g/L et 150g/L de biomasse, et de la lutéine à des taux généralement entre 0,1 et 5% de la matière sèche. La biomasse ainsi produite est particulièrement bien adaptée à l'alimentation de rotifères destinés à la pisciculture, notamment pour l'élevage du bar et de la daurade. Elle est également utilisée comme nutraceutique pour ses propriétés immunostimulantes et détoxifiantes. On peut citer comme exemple de ces souches les souches *Chlorella* FCC 2, les souches *Chlorella* sp. FCC 1553 et FCC 1520.

Les souches du genre *Scenedesmus,* notamment des espèces *Scenedesmus obliquus, Scenedesmus* sp. et *Scenedesmus abundans* en conditions de mixotrophie selon l'invention produisent de l'ALA, de l'acide oléique et de la lutéine. Les souches donnent généralement un rendement en biomasse compris entre 30 et 100 g/L, avec un taux de lipides généralement entre 10 et 60% en matière sèche. L'acide alpha linoléique ou ALA représente généralement entre 10 et 50% des acides gras totaux, et l'acide oléique représente généralement entre 25 et 50% des acides gras totaux, la lutéine représente généralement entre 0,1 à 5% de la matière sèche. On peut citer comme exemple de ces souches les souches *Scenedesmus obliquus* FCC 4, *Scenedesmus* sp. FCC 1483 et *Scenedesmus abundans* FCC 23.

Les souches du genre *Tetraselmis,* notamment *Tetraselmis* sp. produisent, en conditions de mixotrophie selon un mode de réalisation de l'invention, de l'EPA et de l'ALA. Généralement, les souches donnent un rendement en biomasse entre 30 et 80 g/L, avec un taux d'acides gras généralement entre 10 et 30% de la matière sèche. L'éicosapentaénoïque (EPA) représente généralement entre 10 à 25% des acides gras, et l'acide $\alpha$-linolénique (ALA), généralement 5 à 20% des acides gras. La biomasse ainsi produite est particulièrement bien adaptée pour une utilisation en aquaculture. On peut citer comme exemple de ces souches la souche *Tetraselmis* sp. FCC 1563.

Les souches du genre *Haematococcus* produisent, en conditions de mixotrophie selon un mode de réalisation de l'invention de l'astaxanthine. Les souches donnent un rendement en biomasse généralement entre 5 et 30 g/L, et en astaxanthine généralement entre 0,1 à 15% de la matière sèche. On peut citer comme exemple de ces souches la souche *Haematococcus* sp. FCC 1643.

Les souches du genre *Nitzschia* produisent, en conditions de mixotrophie selon un mode de réalisation de l'invention, de l'EPA et de la fucoxanthine. Les souches généralement donnent un rendement en biomasse compris entre 40 et 120 g/L, un rendement en lipides entre 10 et 50% de la matière sèche, en acide éicosapentaénoïque (EPA), entre 15 et 50% des acides gras et en fucoxanthine, entre 0,1 et 5% de la matière sèche. On peut citer comme exemple de ces souches les souches *Nitzschia* sp. FCC 1687.

Dans la présente invention, les souches qui sont cultivées en conditions mixotrophes, en particulier en présence d'un éclairement variable et/ou discontinu, notamment sous forme de flashs, permettent de produire des molécules d'intérêt, notamment des lipides et/ou des pigments. De manière générale, la culture de ces souches appartenant aux genres *Schizochytrium, Aurantiochytrium, Crypthecodinium, Scenedesmus* et *Nitzschia,* qui produisent un lipide et un pigment en conditions d'hétérotrophie, ne produit aucun ou très peu de pigment. De plus, des quantités de biomasse obtenues en mode mixotrophe pour ces souches, selon certains modes de réalisation de l'invention, sont égales ou même supérieures (par exemple, approximativement supérieures de 10 à 18%) aux quantités obtenues en conditions d'hétérotrophie. Par conditions d'hétérotrophie, on entend des conditions de culture avec un milieu de culture identique, mais en l'absence de lumière.

L'invention a ainsi pour objet un procédé de culture de protistes, en mode mixotrophe, en particulier en présence d'un éclairement variable ou discontinu au cours du temps, par exemple sous forme de flashs, notamment en vue de produire des acides gras poly-insaturés ou mono-insaturés, et/ou des caroténoïdes, notamment la lutéine, la fucoxanthine, l'astaxanthine, la canthaxanthine et le $\beta$-carotène.

Selon un aspect préféré, ladite biomasse comprend une teneur en acide(s) gras d'intérêt dans la phase grasse d'au moins 10 %, de préférence au moins 20 %, plus préférentiellement au moins 30 %.

Ladite biomasse peut également comprendre une teneur en pigment(s) d'intérêt dans la phase grasse d'au moins 0,01 %, de préférence au moins 0,1 %, plus préférentiellement au moins 0,5 %.

De manière avantageuse, le procédé selon l'invention comprend en outre au moins une étape de récupération des molécules d'intérêt à partir de la biomasse produite. Par exemple, le procédé selon l'invention peut comprendre en outre au moins une étape de récupération de la matière hydrophobe (qui comporte des lipides et/ou des pigments) et, éventuellement, au moins une étape d'extraction des acides gras, notamment de l'EPA et/ou du DHA et/ou de l'ARA et/ou de l'ALA, et/ou de l'acide oléique et/ou au moins une étape d'extraction des pigments, notamment la lutéine, la fucoxanthine, l'astaxanthine, la zéaxanthine, la canthaxanthine, l'échinénone, le bêta-carotène et la phoenicoxanthine de cette matière hydrophobe

La récupération des molécules d'intérêt peut être effectuée par des procédés conventionnels.

En particulier, lorsque ladite molécule d'intérêt est un acide gras, le procédé selon l'invention comprend éventuellement, en outre, au moins une étape d'extraction de cet acide gras à partir desdits lipides. Les méthodes d'extraction sélective des lipides dont l'EPA, l'ARA et le DHA, sont connues de l'homme du métier et sont, par exemple, décrites par [Bligh, E.G. et Dyer, W.J. (1959); A rapid method of total lipid extraction and purification, Can. J. Biochem. Physiol., 37:911-917].

L'invention est illustrée de manière non limitative par les exemples suivants.

## EXEMPLES

### Exemple 1

La production d'une huile riche en DHA et en canthaxanthine par la souche FCC 1324 du genre *Aurantiochytrium* est décrite ci-dessous.

Etape a) :

Les cultures *d'Aurantiochytrium* sont réalisées dans des fermenteurs (bioréacteurs) de 1 à 2 L utiles avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26 °C. L'agitation est réalisée grâce à 3 mobiles d'agitation placés sur l'arbre selon la configuration de Rushton (hélices tripales à pompage descendant). La pression d'oxygène dissous est régulée dans le milieu tout au long de la culture, par la vitesse d'agitation (250-600 t/min), le débit d'air (0,25-1 vvm), voire le débit d'oxygène (0,1-0,5 vvm). Les paramètres de régulation, intégrés dans l'automate de supervision, permettent de maintenir une $pO_2$ constante à 15 %. Le bioréacteur est équipé d'un système luminaire interne fixé sur les contre-pales. L'intensité, ainsi que les cycles de lumière, sont contrôlés par un automate dédié et supervisé par station informatique. La culture est illuminée avec 60 flashs par heure, chaque flash ayant une durée de 20 secondes et une intensité de 100 $\mu$mol. m$^{-2}$. s$^{-1}$.

Les réacteurs sont inoculés avec une pré-culture réalisée sur table d'agitation (140 t/min) en enceinte thermostatée (26 °C) et éclairée entre 100 et 200 $\mu$E. Pré-cultures et cultures en bioréacteurs sont réalisées dans le milieu Verduyn modifié (sels marins 15 g/L, $(NH_4)_2SO_4$ 3g/L, $KH_2PO_4$ 1 g/L, $MgSO_4 \cdot 7H_2 0$, 0,5 g/L, $Na_2EDTA$ 24 mg/L, $ZnSO_4 \cdot 7H_2O$ 3 mg/L, $MnCl_2 \cdot 2H_2O$ 3 mg/L, $Na_2MoO_4 \cdot 2H_2O$ 0,04 mg/L, FeSO4$\cdot 7H_2O$ 10mg/L, pantothénate 3,2 mg/L, hydrochlorure de thiamine 9,5mg/L, vitamine B12 0,15 mg/L). Le substrat carboné utilisé est du glucose à des concentrations entre 60 et 200 g/L.

L'alimentation en continu avec du milieu frais est réalisée avec un taux de dilution d'environ 0,08 à 0,15 h$^{-1}$ et avec un milieu concentré entre 10 et 15 fois la concentration initiale de chaque élément. L'homme du métier saura déterminer comment mettre en oeuvre la culture continue et calculer le débit d'alimentation, ainsi que déterminer quand le régime permanent est atteint.

Une fois le régime permanent atteint, le soutirage de ce fermenteur continu peut servir à alimenter le fermenteur de maturation de l'étape b).

Etape b) :

Les cultures sont réalisées dans des fermenteurs (bioréacteurs) de 10 à 20 L utiles avec automates dédiés et supervision par station informatique. Les systèmes de régulation ainsi que le paramétrage de ces derniers sont, en tout point, similaires à ceux de l'étape a).

Les réacteurs sont inoculés avec environ 50 % du volume total de culture avec le soutirage de l'étape a) ; et en même temps, la cuve est également alimentée au même débit avec un milieu concentré 2 fois, et qui permet d'obtenir la composition finale suivante : sels marins 15 g/L ; glucose entre 60 et 120 g/L; $(NH_4)_2SO_4$ à 0,8 gL; $KH_2PO_4$ 1 g/L ; $MgSO_4 \cdot 7H_2 0$, 0,5 g/L, $Na_2EDTA$ 24 mg/L, $ZnSO_4 \cdot 7H_2O$ 3 mg/L, $MnCl_2 \cdot 2H_2O$ 3 mg/L, $Na_2MoO_4 \cdot 2H_2O$ 0,04 mg/L, FeSO4$\cdot 7H_2O$ 10mg/L, pantothénate 3,2 mg/L, hydrochlorure de thiamine 9,5 mg/L, vitamine B12 0,15 mg/L.

La culture est illuminée avec 60 flashs par heure, chaque flash ayant une durée de 20 secondes et une intensité de 500 $\mu$mol. m$^{-2}$. s$^{-1}$.

Suivi des cultures :

La concentration en biomasse totale est suivie par mesure de la masse sèche (filtration sur filtre GF/F, Whatman, puis séchage en étuve, 105 °C, pendant 24h minimum avant pesée).

Concernant la quantification des lipides totaux, environ 10$^8$ cellules ont été extraites. Les méthodes d'extraction des lipides sont connues de l'homme du métier.

Pour la quantification des caroténoïdes, et notamment la canthaxanthine, 10$^8$ cellules ont été extraites. Les méthodes d'extraction et d'analyse des caroténoïdes, dont la canthaxanthine, sont connues de l'homme du métier.

**Tableau 4** : Résultats (n=3)

| Masse Sèche (g/L) | Lipides totaux (% de la MS) | % DHA | Canthaxanthine (mg/g de MS) |
|---|---|---|---|
| 155 +/- 3,3 | 55 +/- 2,8 | 18,0 +/- 3,0 | 2,1 +/- 0,3 |

Le Tableau 4 représente des données comparatives pour la production d'huile riche en DHA par *Aurantiochytrium* selon le procédé de l'invention (découplé) et discontinu, semi-continu et continu. Les valeurs citées pour les procédés discontinu, semi-continu et continu sont, à titre prédictif sur une année, basées sur nos expériences précédentes avec cette souche et les autres souches similaires en culture discontinu, semi-continu et continu.

**Tableau 5**

| OPERATION UNITAIRE | BATCH | FED-BATCH | CONTINU | DECOUPLE |
|---|---|---|---|---|
| **Biomasse produite** (pourcentage relatif par rapport à la biomasse produite en mode découplé) | 5,8 % | 29 % | 84,3 % | 100 % |
| Effluents pendant concentration | 1280 m$^3$/an | - | 9.690 m$^3$/an | - |
| | 3,9 m$^3$/jour | | 29,4 m$^3$/jour | |
| Effluents pendant séchage (eau) | 280 tonnes/an | 1400 tonnes/an | 3990 tonnes/an | 4810 tonnes/an |
| | 0,85 tonnes/jour | 4,2 tonnes/jour | 13,3 tonnes/jour | 14,6 tonnes/jour |
| **Ratio Kg effluents / Kg de biomasse produite** *Hors nettoyage* | **39 L** | **7 L** | **24 L** | **7 L** |

**Exemple 2**

La production en continu d'une biomasse à haute densité cellulaire pauvre en matière grasse (5 à 10%) puis l'accumulation d'acides gras par maturation de cette biomasse

Etape a): Culture continue pour la production de biomasse

Les cultures d'Aurantiochytrium sont réalisées dans des réacteurs de 1 à 2 L de volume utile avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26 °C. L'agitation est réalisée grâce à 3 mobiles d'agitation : 1 turbine Rushton à 6 pâles droites positionnée à l'extrémité inférieure de l'arbre d'agitation au-dessus du sparger et 2 hélices tripâles placées sur l'arbre d'agitation (distance inter-mobile = 1.5 X le diamètre d'une hélice).

La pression d'oxygène dissous est régulée dans le milieu tout au long de la culture, par la vitesse de rotation de l'arbre d'agitation (250-600 t/min), la ventilation par l'air (0,25-1 vvm) ou d'oxygène (0,1-0,5 vvm). Les paramètres de régulation, intégrés dans l'automate de supervision, permettent de maintenir une pO$_2$ constante à 15 %.

Le fermenteur est équipé d'un système luminaire interne fixé sur les contre-pâles.

L'intensité et la fréquence des cycles d'illumination sont contrôlées par un automate dédié et supervisé par station informatique. La culture est illuminée avec 60 flashs par heure, chaque flash ayant une durée de 20 secondes et une intensité de 100 µmol. m-2. s-1.

La pré-culture est réalisée sur table d'agitation (140 t/min) en enceinte thermostatée (26 °C) et éclairée entre 100 et 200 µE. Le réacteur est inoculé à 1% à partir de la pré-culture et conduit en mode discontinu.

Une fois le substrat contenu dans le milieu initial consommé et la densité cellulaire souhaitée atteinte, le soutirage du milieu de culture d'une part et l'apport de la solution d'alimentation d'autre part sont mis en route. Le régime permanent est atteint après au moins 5 temps de séjour. Une fois le régime permanent stabilisé, les paramètres opératoires (débit de ventilation, puissance dissipée, débit d'alimentation, pH, volume de moût), la concentration résiduelle en substrat dans le moût, ainsi que la concentration et la composition macromoléculaire de la biomasse produite sont constants.

Pour la souche FCC 1324, la composition de la solution d'alimentation choisie et le taux de dilution sélectionné permettent de produire un volume de moût équivalent à 2,4 fois le volume du fermenteur chaque jour.

Le régime est considéré stable au bout de 50 heures de culture. Une fois le régime stabilisé, la concentration résiduelle en glucose dans le moût se stabilise vers 0 g/L, la densité cellulaire se stabilise autour de 3x10$^9$ cellules/mL, ce qui correspond à environ 65 g/L de biomasse, la teneur en matière grasse dans la biomasse se stabilise autour de 5% (gMatière Grasse/gMatière Sèche). La fermentation en mode continu a été maintenue pour une durée totale de 890 heures, dont 815 heures en régime stabilisé.

Pour cet exemple le taux de dilution par la solution d'alimentation est fixé à la moitié du taux de croissance maximum de la souche sur le milieu utilisé et dans les conditions de culture utilisées.

Le débit de soutirage du milieu est adapté afin de prendre en compte le débit de la solution d'alimentation mais aussi le volume de liquide correcteur de pH.

La composition du milieu de culture pour le démarrage en batch de la culture est détaillée dans le Tableau 1.

La solution d'alimentation de la culture continue contient 15g/L de sel de mer commercial (ex Sels INSTANT OCEAN), 110 g/L de glucose, et contient des concentrations en oligo-éléments, macro-éléments et vitamines équivalentes à environ 3 fois celles contenues dans le milieu utilisé pour la pré-culture et la culture discontinue. Des autres compositions pour la solution d'alimentation sont possible, comme détaillée dans le Tableau 1.

La concentration en biomasse totale est suivie par mesure de la masse sèche (filtration sur filtre GF/F, Whatman, puis séchage en étuve, 105 °C, pendant 24h minimum avant pesée).

Concernant la quantification des lipides totaux, environ $10x^8$ cellules ont été extraites. Les méthodes d'extraction des lipides sont connues de l'homme du métier.

Pour la quantification des caroténoïdes, et notamment la canthaxanthine, $10x^8$ cellules ont été extraites. Les méthodes d'extraction et d'analyse des caroténoïdes, dont la canthaxanthine, sont connues de l'homme du métier.

**Tableau 6.** Caractéristiques du régime permanent obtenu en culture continue.

| Glucose résiduel | $0.1 \pm 0.02$ | g/L |
|---|---|---|
| Densité cellulaire | 3.109 +/- 0,5.109 | cellules/mL |
| Masse sèche | 65 +/- 5 | g/L |
| Teneur en acide gras | 7% +/- 3% | % |

Etape b) de maturation

Le moût produit en culture continue présente donc une haute densité cellulaire d'une biomasse contenant très peu de lipides (5 à 10%). Cette biomasse est transférée dans une cuve de maturation dans des conditions de culture où elle accumule très rapidement des acides gras. (65 G/L en 24 heures). Ceci permet de produire une forte concentration de biomasse riche en acide gras.

Dans cet exemple, plusieurs essais de maturation sont effectués à partir du moût issu du fermenteur conduit en mode chemostat. Les cultures sont réalisées dans des fermenteurs (bioréacteurs) de 3 à 5 L utiles avec automates dédiés et supervision par station informatique. Les systèmes de régulation ainsi que le paramétrage de ces derniers sont, en tout point, similaires à ceux de l'étape a).

Dans le cas des essais de maturation décrits dans cet exemple, le volume prélevé dans la culture continue est prélevé en une fois. Dans le cas d'une fermentation industrielle, ce transfert se fait progressivement par le soutirage du milieu de culture.

Dans la cuve de maturation, la solution nutritive avec un ratio C/N/P de 530 :11 :1 est progressivement ajoutée au volume prélevé.

La solution nutritive est ajoutée dans la cuve de maturation à chaque fois que la source de carbone (ici le glucose) est épuisée.

Le suivi de la culture discontinue alimentée, réalisée en cuve de maturation, est réalisé selon des protocoles en tout point identiques à ceux de l'étape i). Les résultats sont donnés dans le Tableau 7.

**Tableau 7 :**

| Matière sèche, acides gras et DHA produits en 24 heures et en 48 heures lors de la maturation à 25°C et pH 6,5 (5 répétitions) | | |
|---|---|---|
| | **24 Heures de culture** | **48 Heures de culture** |
| Matière Sèche | 160 g/L (+/- 2g/l) | 185 g/l (+/-5g/l) |
| Acides Gras | 67 g/L (+/-2g/l) | 85 g/l (+/-5g/l) |
| DHA | 16,5 g/L(+/-0,5g/l) | 21 g/l (+/-2g/l) |
| Astaxanthine | 0,05 mg/g MS (+/-0,01) | 0,07 mg/g MS (+/-0,01) |

**Exemple 3.**

La production de biomasse utilisable en pisciculture ou pour la production de lutéine par la souche FCC 1520 du genre *Chlorella* est décrite ci-dessous.

Etape a) :

Les cultures de *Chlorella* sont réalisées dans des fermenteurs (bioréacteurs) de 1 à 2 L utiles avec automates dédiés et supervision par station informatique. Le système est régulé en pH via l'ajout de base (solution d'hydroxyde de sodium à 2N) et/ou d'acide (solution d'acide sulfurique à 1N). La température de culture est fixée à 26 °C. L'agitation est réalisée grâce à 3 mobiles d'agitation placés sur l'arbre selon la configuration de Rushton (hélices tripales à pompage descendant). La pression d'oxygène dissous est régulée dans le milieu tout au long de la culture, par la vitesse d'agitation (250-600 t/min), le débit d'air (0,25-1 vvm), voire le débit d'oxygène (0,1-0,5 vvm). Les paramètres de régulation, intégrés dans l'automate de supervision, permettent de maintenir une $pO_2$ constante à 15 %. Le bioréacteur est équipé d'un système luminaire interne fixé sur les contre-pales. L'intensité, ainsi que les cycles de lumière, sont contrôlés par un automate dédié et supervisé par station informatique. La culture est illuminée avec 30 flashs par heure, chaque flash ayant une durée de 60 secondes et une intensité de 50 à 100 $\mu$mol. m$^{-2}$. s$^{-1}$.

Les réacteurs sont inoculés avec une pré-culture réalisée sur table d'agitation (140 t/min) en enceinte thermostatée (26 °C) et éclairée entre 100 et 200 $\mu$E. Pré-cultures et cultures en bioréacteurs sont réalisées dans le milieu suivant : Glucose 20 g/L ; KNO3 2 g/L ; NaH2PO4 0,54 g/L ; Na2HPO4,12H2O 0,179 g/L ; MgSO4,7H2O 0,2465 g/L ; CaCl2,2H2O 0,0147 g/L ; Extrait de levure 0,25 g/L; FeSO4,7H2O 0,01035 g/L; H3BO3 0,000061 g/L; MnSO4,H2O 0,000169 g/L; ZnSO4,7H2O 0,000287 g/L; CuSO4,5H2O 0,0000025 g/L ; (NH4)6MoO24,4H2O 0,0000125 g/L ; Thiamine Hcl (vitamine B1) 0,2 mg/L ; Biotine (Vitamine H) 0,001 mg/L ; Cyanocobalamine (vitamine B12) 0,001 mg/L.

L'alimentation en continu avec du milieu frais est réalisée avec un taux de dilution d'environ 0,08 à 0,15 h$^{-1}$ et avec le milieu suivant : Glucose 224,55 g/L; KNO3 22,45 g/L; MgSO4,7H2O 0,165 g/L; CaCl2,2H2O 2,77 g/L; FeSO4,7H2O 0,17 g/L; H3BO3 0,0015 g/L; MnSO4,H2O 0,0042 g/L; ZnSO4,7H2O 0,0072 g/L ; CuSO4,5H2O 0,0000625 g/L ; (NH4)6MoO24,4H2O 0,0003125 g/L ; Thiamine Hcl (vitamine B1) 2,24 mg/L ; Biotine (Vitamine H) 0,0112 mg/L ; Cyanocobalamine (vitamine B12) 0,0112 mg/L. L'homme du métier saura déterminer comment mettre en oeuvre la culture continue et calculer le débit d'alimentation, ainsi que déterminer quand le régime permanent est atteint.

Une fois le régime permanent atteint, le soutirage de ce fermenteur continu peut servir à alimenter le fermenteur de maturation de l'étape b).

Etape b) :

Les cultures sont réalisées dans des fermenteurs (bioréacteurs) de 10 à 20 L utiles avec automates dédiés et supervision par station informatique. Les systèmes de régulation ainsi que le paramétrage de ces derniers sont, en tout point, similaires à ceux de l'étape a).

Les réacteurs sont inoculés avec environ 50 % du volume total de culture avec le soutirage de l'étape a) ; et en même temps, la cuve est également alimentée au même débit avec un milieu concentré 2 fois, et qui permet d'obtenir la composition finale suivante : Glucose 500 g/L ; KNO3 50 g/L ; NaH2PO4 13,5 g/L ; Na2HPO4,12H2O 4,47 g/L ; MgSO4,7H2O 6,1625 g/L ; CaCl2,2H2O 0,3675 g/L; FeSO4,7H2O 0,086 g/L; H3BO3 0,061 g/L; MnSO4,H2O 0,169 g/L ; ZnSO4,7H2O 0,287 g/L ; CuSO4,5H2O 0,0025 g/L ; (NH4)6MoO24,4H2O 0,0125 g/L ; Thiamine Hcl (vitamine B1) 5 mg/L ; Biotine (Vitamine H) 0,025 mg/L ; Cyanocobalamine (vitamine B12) 0,025 mg/L.

La culture est illuminée avec 30 flashs par heure, chaque flash ayant une durée de 20 secondes et une intensité de 100 $\mu$mol. m$^{-2}$. s$^{-1}$.

Suivi des cultures :

La concentration en biomasse totale est suivie par mesure de la masse sèche (filtration sur filtre GF/F, Whatman, puis séchage en étuve, 105 °C, pendant 24h minimum avant pesée).

Pour la quantification des caroténoïdes, et notamment la lutéine, $10^8$ cellules ont été extraites. Les méthodes d'extraction et d'analyse des caroténoïdes, dont la lutéine, sont connues de l'homme du métier.

**Tableau 8** : Résultats (n=3)

| Masse Sèche (g/L) | Canthaxanthine (mg/g de MS) |
|---|---|
| 130 +/- 5 | 5,1 +/- 0,1 |

**Revendications**

1. Procédé de production de biomasse de cellules d'organismes unicellulaires eucaryotes capables de croître en utilisant la lumière et une source carbonée organique, **caractérisé en ce qu'**il consiste en :

   a) la culture des cellules en mode continu en conditions de mixotrophie ou d'hétérotrophie dans un fermenteur, puis
   b) l'alimentation en continu et successivement de n fermenteurs fonctionnant en mode semi-continu, n étant un nombre entier égal ou supérieur à 2, par les cellules produites dans l'étape a) et leur culture en conditions de mixotrophie

   et **en ce que** la culture des cellules en conditions de mixotrophie s'effectue dans des conditions d'éclairement discontinu et/ou variable au cours du temps,
   et **en ce que** l'éclairement présente des variations d'intensité dont l'amplitude est comprise entre 50 et 1000 $\mu$mol.m-2.s-1, ces variantions ayant lieu entre 2 et 3600 fois par heure.

2. Procédé selon la revendication 1, **caractérisé en ce que** l'éclairement présente des variations d'intensité dont l'amplitude est comprise entre 50 et 400 $\mu$mol. m-2.s-1, ces variations ayant lieu entre 2 et 200 fois par heure.

3. Procédé selon l'une quelconque des revendications 1 ou 2, **caractérisé en ce que** l'éclairement est sous forme de flashs.

4. Procédé selon l'une quelconque des revendications 1 à 3, **caractérisé en ce que** la culture dans l'étape a) se fait en présence des flashs, ayant une intensité de 50 à 200 $\mu$mol. m-2. s-1 et une durée d'environ 1/10ème de seconde à cinq minutes, de préférence entre une seconde et une minute, et entre 2 et 3600, de préférence, entre 2 et 360 flashs par heure.

5. Procédé selon l'une quelconque des revendications 1 à 4, **caractérisé en ce que** la culture dans l'étape b) se fait en présence des flashs, ayant une intensité de 50 à 2000 $\mu$mol. m-2. s-1 et une durée d'environ 1/10ème de seconde à cinq minutes, de préférence entre une seconde et une minute et, entre 2 et 3600, de préférence, entre 2 et 360 flashs par heure.

6. Procédé selon l'une quelconque des revendications 1 à 5, **caractérisé en ce que** la culture dans les étapes a) et/ou b) est effectuée en présence d'un substrat carboné organique à une concentration de 5 mM à 1,1 M, de préférence de 50 mM à 800 mM.

7. Procédé selon l'une quelconque des revendications 1 à 6, **caractérisé en ce que** les organismes unicellulaires eucaryotes sont choisis parmi les protistes marins ou d'eau douce photosynthétiques.

8. Procédé selon l'une des revendications 1 à 6, **caractérisé en ce que** les organismes unicellulaires eucaryotes sont des protistes mixotrophes choisis parmi les espèces du genre suivant : *Schizochytrium, Thraustochytrium, Odontella, Phaeodactylum,* Nannochloris, *Crypthecodinium, Monodus, Nannochloropsis, Isochrysis, Euglena, Cyclotella, Nitzschia, Aurantiochytrium, Scenedesmus* et/ou *Tetraselmis.*

9. Procédé selon l'une des revendications 1 à 6, **caractérisé en ce que** les organismes unicellulaires eucaryotes sont des protistes mixotrophes choisis parmi les espèces *Chlorella* et *Haematococcus.*

10. Procédé selon l'une quelconque des revendications 1 à 9, **caractérisé en ce qu'**il comprend en outre au moins une étape de récupération de la biomasse issue de l'étape b).

11. Procédé selon la revendication 10, **caractérisé en ce que** ladite biomasse comprend au moins une molécule d'intérêt choisie parmi un alcool, un acide organique, un acide gras, un polysaccharide, un terpène tel qu'un botryococcène, un pigment tel qu'un caroténoïde, un acide aminé, une enzyme, une vitamine, un antibiotique, un composé à activité pharmacologique tel qu'une protéine exogène ou recombinante et un pigment.

12. Procédé selon la revendication 11, **caractérisé en ce que** ladite molécule d'intérêt est choisie parmi l'acide éicosapentaénoïque (EPA) et l'acide docosahexaénoïque (DHA), l'acide arachidonique (ARA), l'acide $\alpha$-linolénique (ALA), l'acide oléique, la fucoxanthine, la canthaxanthine, l'astaxanthine, la lutéine et le bêta-carotène.

**13.** Procédé selon l'une quelconque des revendications 1 à 12, **caractérisé en ce qu'**il comprend en outre au moins une étape de récupération des lipides et/ou des pigments, éventuellement, au moins une étape d'extraction de l'EPA et/ou du DHA et/ou de l'ARA et/ou des pigments à partir desdits lipides.

**14.** Procédé selon l'une quelconque des revendications 1 à 13, **caractérisé en ce que** l'étape b) est réalisée entre 15 et 24°C.

**Patentansprüche**

**1.** Verfahren für die Produktion von Biomasse aus Zellen eukaryotischer einzelliger Organismen, die bei Nutzung von Licht und einer organischen kohlenstoffhaltigen Quelle zum Wachstum imstande sind, **dadurch gekennzeichnet, dass** es besteht in:

a) der Kultur der Zellen im kontinuierlichen Modus unter Mixotrophie- oder Heterotrophiebedingungen in einem Fermenter, dann

b) der kontinuierlichen und aufeinanderfolgenden Versorgung von *n* Fermentern, die im halbkontinuierlichen Modus arbeiten, wobei n eine Ganzzahl gleich oder größer als 2 ist, mit den in Schritt a) produzierten Zellen und deren Kultur unter Mixotrophiebedingungen

und dass die Kultur der Zellen unter Mixotrophiebedingungen unter Bedingungen diskontinuierlicher und/oder variabler Beleuchtung im Zeitverlauf erfolgt,
und dass die Beleuchtung Intensitätsschwankungen aufweist, deren Amplitude zwischen 50 und 1000 $\mu$mol.m-2.s-1 inklusive liegt, wobei diese Schwankungen zwischen 2 und 3600 Mal pro Stunde stattfinden.

**2.** Verfahren nach Anspruch 1, **dadurch gekennzeichnet, dass** die Beleuchtung Intensitätsschwankungen aufweist, deren Amplitude zwischen 50 und 400 $\mu$mol.m-2.s-1 inklusive liegt, wobei diese Schwankungen zwischen 2 und 200 Mal pro Stunde stattfinden.

**3.** Verfahren nach einem der Ansprüche 1 oder 2, **dadurch gekennzeichnet, dass** die Beleuchtung in Form von Blitzen erfolgt.

**4.** Verfahren nach einem der Ansprüche 1 bis 3, **dadurch gekennzeichnet, dass** die Kultur in Schritt a) bei Anwesenheit von Blitzen mit einer Intensität von 50 bis 200 $\mu$mol.m-2.s-1 und einer Dauer von zirka 1/10 Sekunde bis fünf Minuten, vorzugsweise zwischen einer Sekunde und einer Minute und zwischen 2 und 3600, vorzugsweise zwischen 2 und 360 Blitzen pro Stunde erfolgt.

**5.** Verfahren nach einem der Ansprüche 1 bis 4, **dadurch gekennzeichnet, dass** die Kultur in Schritt b) bei Anwesenheit von Blitzen mit einer Intensität von 50 bis 2000 $\mu$mol.m-2.s-1 und einer Dauer von zirka 1/10 Sekunde bis fünf Minuten, vorzugsweise zwischen einer Sekunde und einer Minute und zwischen 2 und 3600, vorzugsweise zwischen 2 und 360 Blitzen pro Stunde erfolgt.

**6.** Verfahren nach einem der Ansprüche 1 bis 5, **dadurch gekennzeichnet, dass** die Kultur in den Schritten a) und/oder b) bei Anwesenheit eines organischen kohlenstoffhaltigen Substrats in einer Konzentration von 5 mM bis 1,1 M, vorzugsweise von 50 mM bis 800 mM, erfolgt.

**7.** Verfahren nach einem der Ansprüche 1 bis 6, **dadurch gekennzeichnet, dass** die eukaryotischen einzelligen Organismen aus den photosynthetischen maritimen oder Süßwasserprotisten ausgewählt sind.

**8.** Verfahren nach einem der Ansprüche 1 bis 6, **dadurch gekennzeichnet, dass** die eukaryotischen einzelligen Organismen mixotrophe Protisten sind, die aus den Spezies der folgenden Gattung ausgewählt sind: *Schizochytrium, Thraustochytrium, Odontella, Phaeodactylum,* Nannochloris, *Crypthecodinium, Monodus, Nannochloropsis, Isochrysis, Euglena, Cyclotella, Nitzschia, Aurantiochytrium, Scenedesmus* und/oder *Tetraselmis.*

**9.** Verfahren nach einem der Ansprüche 1 bis 6, **dadurch gekennzeichnet, dass** die eukaryotischen einzelligen Organismen mixotrophe Protisten sind, die aus den Spezies *Chlorella* und *Haematococcus* ausgewählt sind.

**10.** Verfahren nach einem der Ansprüche 1 bis 9, **dadurch gekennzeichnet, dass** es ferner mindestens einen Rückgewinnungsschritt der Biomasse aus Schritt b) umfasst.

11. Verfahren nach Anspruch 10, **dadurch gekennzeichnet, dass** die Biomasse mindestens ein interessierendes Molekül umfasst, das aus einem Alkohol, einer organischen Säure, einer Fettsäure, einem Polysaccharid, einem Terpen wie ein Botryococcen, einem Pigment wie ein Carotinoid, einer Aminosäure, einem Enzym, einem Vitamin, einem Antibiotikum, einer Verbindung mit pharmakologischer Aktivität wie ein exogenes oder rekombinantes Protein und einem Pigment ausgewählt ist.

12. Verfahren nach Anspruch 11, **dadurch gekennzeichnet, dass** das interessierende Molekül aus der Eicosapentaensäure (EPA) und der Docosahexaensäure (DHA), der Arachidonsäure (ARA), der α-Linolensäure (ALA), der Ölsäure, dem Fucoxanthin, dem Canthaxanthin, dem Astaxanthin, dem Lutein und dem Beta-Carotin ausgewählt ist.

13. Verfahren nach einem der Ansprüche 1 bis 12, **dadurch gekennzeichnet, dass** es ferner mindestens einen Rückgewinnungsschritt der Lipide und/oder der Pigmente, eventuell einen Extraktionsschritt der EPA und/oder der DHA und/oder der ARA und/oder der Pigmente aus den Lipiden umfasst.

14. Verfahren nach einem der Ansprüche 1 bis 13, **dadurch gekennzeichnet, dass** Schritt b) zwischen 15 und 24 °C durchgeführt wird.

**Claims**

1. A method for producing biomass from cells of eukaryotic unicellular organisms capable of growing using light and an organic carbon-containing source, **characterized in that** it consists of:

   a) the culture of the cells in continuous mode under mixotrophic or heterotrophic conditions in a fermenter, then
   b) the continuous and successively feeding of *n* fermenters operating in semi-continuous mode, *n* being an integer equal to or greater than 2, with the cells produced in step a) and their culture under mixotrophic conditions

   and **in that** the culture of the cells under mixotrophic conditions is carried out under conditions of discontinuous and/or variable illumination over time,
   and **in that** the illumination has variations in intensity the amplitude of which is comprised between 50 and 1,000 $\mu$mol.m-2.s-1, these variations taking place between 2 and 3,600 times per hour.

2. Method according to claim 1, **characterized in that** the illumination has variations in intensity the amplitude of which is comprised between 5 and 1,000 $\mu$mol.m-2.s-1, these variations taking place between 2 and 200 times per hour.

3. Method according to any one of claims 1 or 2, **characterized in that** the illumination is in the form of flashes.

4. Method according to any one of claims 1 to 3, **characterized in that** the culture in step a) is carried out in the presence of flashes having an intensity of 50 to 200 $\mu$mol.m-2.s-1 and a duration of approximately 1/10th of a second to five minutes, preferably between a second and one minute, and between 2 and 3,600, preferably between 2 and 360 flashes per hour.

5. Method according to any one of claims 1 to 4, **characterized in that** the culture in step b) is carried out in the presence of flashes having an intensity of 50 to 2,000 $\mu$mol.m-2.s-1 and a duration of approximately 1/10th of a second to five minutes, preferably between a second and one minute, and between 2 and 3,600, preferably between 2 and 360 flashes per hour.

6. Method according to any one of claims 1 to 5, **characterized in that** the culture in steps a) and/or b) is carried out in the presence of an organic carbon-containing substrate at a concentration from 5 mM to 1.1 M, preferably from 50 mM to 800 mM.

7. Method according to any one of claims 1 to 6, **characterized in that** the eucaryotic unicellular organisms are chosen from marine or fresh-water photo synthetic protists.

8. Method according to one of claims 1 to 6, **characterized in that** the eucaryotic unicellular organisms are mixotrophic protists chosen from the species of the following genera: *Schizochytrium, Thraustochytrium, Odontella, Phaeodac-*

*tylum, Nannochloris, Crypthecodinium, Monodus, Nannochloropsis, Isochrysis, Euglena, Cyclotella, Nitzschia, Aurantiochytrium, Scenedesmus* and/or *Tetraselmis.*

9. Method according to one of claims 1 to 6, **characterized in that** the eucaryotic unicellular organisms are mixotrophic protists chosen from the species *Chlorella* and *Haematococcus.*

10. Method according to any one of claims 1 to 9, **characterized in that** it further comprises at least one step of recovery of the biomass resulting from step b).

11. Method according to claim 10, **characterized in that** said biomass comprises at least one molecule of interest chosen from an alcohol, an organic acid, a fatty acid, a polysaccharide, a terpene such as botryococcene, a pigment such as carotenoid, an amino acid, an enzyme, a vitamin, an antibiotic, a compound with pharmacological activity such as exogenous or recombinant protein and a pigment.

12. Method according to claim 11, **characterized in that** said molecule of interest is chosen from eicosapentaenoic acid (EPA) and docosahexaenoic acid (DHA), arachidonic acid (ARA), $\alpha$-linolenic acid (ALA), oleic acid, fucoxanthin, canthaxanthin, astaxanthin, lutein and beta-carotene.

13. Method according to any one of claims 1 to 12, **characterized in that** it further comprises at least one step of recovery of lipids and/or pigments, optionally at least one step of extraction of EPA and/or DHA and/or ARA and/or pigments from said lipids.

14. Method according to any one of claims 1 to 13, **characterized in that** step b) is carried out between 15 and 24 °C.

| Cryotube 1 mL | Erlen 20 mL | Erlen 100 mL | Fermenteur 40 L | Fermenteur 4000 L |

Taux d'inoculation

1%

1%

1%

1%

5%

Fig. 1

Durée de culture     24 h     24 h à 72h     24 h          Continu

Temps d'inoculation     0 h          24 h          48 h          72 h          96 h
Taux d'inoculation      50%          50%           50%           50%           50%

Temps de culture     24h à 120h     48h à 144h     72h à 168h     96h à 192h     120h à 216h

| Fermenteur de maturation 1 20000L | Fermenteur de maturation 2 20000L | Fermenteur de maturation 3 20000L | Fermenteur de maturation 4 20000L | Fermenteur de maturation 5 20000L |

EP 3 019 593 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6156570 A
- WO 2003020919 A
- US 20090263889 A
- WO 2012074502 A
- US 2009209014 A
- FR 1057380
- FR 1353641

**Littérature non-brevet citée dans la description**

- **YANG, C. et al.** *Biochemical Engineering Journal,* 2000, vol. 6, 87-102
- Culture Collection of Algae and Protozoa. Scottish Association for Marine Science, Dunstaffnage Marine Laboratory
- *Folia Microbiol (Praha),* 02 Avril 2013
- Culture Collection of Algae and Protozoa. Scottish Association for Marine Science
- **YEN HW ; ZHANG Z.** *Bioresource Technology,* Octobre 2011, vol. 102 (19), 9279-81
- **BHOSALE P ; GADRE RV.** *Lett Appl Microbiol.,* 2002, vol. 34 (5), 349-53
- **BLIGH, E.G. ; DYER, W.J.** A rapid method of total lipid extraction and purification. *Can. J. Biochem. Physiol.,* 1959, vol. 37, 911-917